(19) Europäisches Patentamt
European Patent Office
Office européen des brevets

(11) EP 0 940 144 A1

(12) EUROPEAN PATENT APPLICATION
published in accordance with Art. 158(3) EPC

(43) Date of publication:
08.09.1999 Bulletin 1999/36

(21) Application number: 97944117.7

(22) Date of filing: 15.10.1997

(51) Int. Cl.$^6$: A61K 47/48

(86) International application number:
PCT/JP97/03710

(87) International publication number:
WO 98/16253 (23.04.1998 Gazette 1998/16)

(84) Designated Contracting States:
AT BE CH DE DK ES FI FR GB GR IE IT LI LU MC NL PT SE

(30) Priority: 15.10.1996 JP 27242596

(71) Applicant: EBARA CORPORATION
Ota-ku Tokyo, 144 (JP)

(72) Inventors:
- FUKAGAWA, Yasuo
Kamakura-shi, Kanagawa 247 (JP)
- MIYA, Akiko
Fujisawa-shi, Kanagawa 251 (JP)

(74) Representative:
Wagner, Karl H., Dipl.-Ing.
WAGNER & GEYER
Patentanwälte
Gewürzmühlstrasse 5
80538 München (DE)

# (54) BIOACTIVE POLYMER PRODUCT

(57) A biologically active polymer product having a biologically active compound moiety immobilized to a polymer substrate through a covalent bond. The biologically active compound moiety such as an antibiotic exerts selective biological activity while being covalently bonded to the polymer substrate. A biologically active polymer product in which two or more biologically active compound moietys are linked to a polymer substrate through a graft chain or a pendant chain.

EP 0 940 144 A1

## Description

TECHNICAL FIELD

This invention relates to a biologically active polymer product, and particularly, it relates to a polymer product wherein an immobilized, biologically active compound moiety exerts selective biological activity while being covalently bonded to a polymer substrate.

BACKGROUND ART

Thus far, in research and development of drugs having biological activities, drugs having greater specificity against targets have been searched for better selective toxicity. On the other hand, with respect to known drugs, they are being used in certain controlled places and technology is being developed to reduce their influence on organisms other than those targeted. On this basis an effort is being made to suppress the adverse influence of residual drugs on the environment to the minimum. For example, a known scheme is that the combination of a harmful-insect attractant and an insecticide is used to terminate only a harmful insect target by attracting it to the insecticide. However, with respect to microorganisms, technology has not yet been developed to use such drugs that attract only the target microorganisms and kill them alone. Therefore, there are no available methods for controlling the target microorganisms, other than to singly use drugs having selective toxicity against them; and thus the residual drugs have been influencing the environment adversely.

Under these circumstances, where raw materials for agriculture, forestry and fishery, and foods are manufactured in large quantities, chemotherapeutics that were originally developed for the emergency treatment of human or animal subjects are used as such, or used after their partial modifications; and organic, synthetic agrochemical with low selectivity of action (insecticides, bactericides, herbicides, oyster-attaching preventers, etc) are used. This not only gives drug-induced sufferings, which can not be ignored, to those other than the targets, but also induces the appearance of tolerant organisms due to the diffusion of the drugs within an environment. A serious danger is thus pointed out that the drugs, which were developed for the emergency treatment of humans, can no longer be employed in their intended therapeutic use. In other words, drug-resistance, especially resistance to a great variety of antibiotics (multiple drug resistance) prevails among the group of non-pathogenic microorganisms that constitutes an overwhelming majority in the ecological system of nature, and it is also amplifying propagation of the multiple drug resistance among pathogenic bacteria, directly or indirectly: such possibility is apprehended. (M. Yoshikawa, Bull. of Jap. Soc. of Microbial Ecology, 10, No. 3, 141-148 (1995).)

In medical treatment also, for example, anticancer drugs are expected to exert useful, selective toxicity effects only on cancer cells. However, when known anticancer drugs are actually administered to human subjects as such, they, as opposed to expectations, act not only on the cancer cells, but also on normal cells to bring undesirable adverse reactions on the patients. This, therefore, necessitates various considerations in their administration. Known as an example to be mentioned is a method of the drug use: in the clinical care of cancer by an anticancer drug, a drug the free diffusion of which is significantly restricted is placed on the vicinity of the affected part, and the drug is allowed to be released slowly and locally so that the adverse reactions of the drug effecting on normal cells can be alleviated. For example, an anticancer drug such as lomustine is sealed in capsules which comprise high-molecular weight polylactic acid that is hydrolyzed non-enzymically in vivo. Alternatively mitomycin C, an antitumor antibiotic, is processed such that it is allowed to be linked to a carboxyl side chain of dextran which is hydrolyzed enzymically in vivo (M. Hashida et al., Chem. Pharm. Bull. 2951 (1982)). These result in controlled-releasing medicinal preparations; and methods for their use in cancer therapy can be mentioned among others. Nevertheless, even in these cases, because the drugs exert anticancer activity by being liberated in vivo, their adverse reactions on normal cells may be alleviated, but can not be eliminated.

In the traditional concept in pharmaceutical sciences, it has been fundamentally believed that drugs can not exert their intrinsic effects if they have been converted to the immobilized state, and that they can do so only in cases where they are used in their free state. Accordingly, no provisions exist in the laws and rules of the world pertaining to the manufacture, the use, the approval, etc. of drugs that deal with drug-immobilized polymer substrate preparations in which low-molecular weight drugs are allowed to be linked to polymer substances so as not to cause liberation of the drugs and which can exert therapeutic effects without involving the liberation. To put it differently, in the case of medicinal preparations where low-molecular weight drugs are immobilized to polymer substances, it has been believed that the liberation of the drugs is an essential prerequisite when they are allowed to act on targets like the controlled-releasing preparations stated above. It can be said that there has been no need to make the controlling laws and regulations, because the drugs have been believed to be totally ineffective if their liberation does not occur.

All drugs (which mean compounds, including from toxic substances to therapeutic agents, that have a wide spectrum of physiological activity) exist in a space or region as their free or dissolved state and move and diffuse freely

within their environments. In pharmaceutical sciences, the fundamental mechanism of action that the drugs reach agents, which are the targets of their use, and exert their characteristic biological activities was therefore an essential prerequisite for use of the drugs. Namely, the target agents were nonmotile and it was the drugs that move to the sites where their effects would manifest. For this reason, when drugs are used, they not only act on the targets, but also have some influence on all the agents coexisting within their environments more or less, which influence is undesirable adverse reactions in many cases: this was unavoidable in principle.

For instance, antimicrobial agents that are generally referred to as "disinfectants," "bactericides," "antimold agents," or the like are necessary commodities in daily life of the present. However, they are deficient in selectivity of biological action; and they are released within their environments because of the drugs' intrinsic solubility and free diffusibility to cause their serious accumulation within the environments, which has been bringing accumulative, adverse effects on humans, animals, and plants over a prolonged period of time.

On one hand, particularly from the standpoint of the direct exclusion or alleviation of toxicity in the living things that are the subjects of treatment such as humans or cattle, a policy has been adopted all over the world in the research, the development, and the use of chemotherapeutics to date: the drugs are selected such that their biological activities are limited to their targets as much as possible and they only bring low adverse reactions at zero or tolerable levels on those other than the targets (high selectivity of action). On the other hand, the drugs that are inevitably diffused and remain within their environments as a result of their use, however, irreversibly destruct the ecological system of the environment in which a wide diversity and variety of organisms exists as mingled and which functions normally while maintaining delicate balance: if viewed from the present standpoint when preservation of the environment has become essential in a broad sense, it can be said that to circumvent this, the high selectivity of drug action has proven to be even more desired than before. For example, the living bodies of humans and animals are where the respective ecological systems of microorganisms peculiar to them are functioning; once the balance of the ecological systems of the microorganisms is lost owing to imprudent drug use, harmful microorganisms that have not become dominant species before multiply, which may cause new serious disorders. An increase in the frequency of onset of opportunistic infections (endogenous infections) can be mentioned as an example of the consequence.

As has been stated above, all the drugs such as bactericides, insecticides, antimold agents, and agrochemical that have been used up till now and that have low selectivity of action, in addition to chemotherapeutics that have considerable selectivity of action diffuse into their environments regardless of their selectivity of action (high or low), since they are used in their free state; and they bring influence, which can not be ignored, on ordinary organisms or cells other than the target organisms, which turns to be the cause of destruction of normal balance of the ecological system.

In these days when the environmental degradation or the adverse reactions of drugs have become a serious public concern, if a novel fundamental prerequisite in pharmaceutical sciences which is the drug use under conditions causing no free diffusion is hypothesized in addition to the conventional, fundamental prerequisite in pharmaceutical sciences which is the drug use under the conditions of free diffusion, and concrete methods for utilization of the drugs are developed, then it can provide a new solution to the problems of environmental contamination and medical treatment: so contemplated the present inventors.

If another expression is employed from the standpoint of the utility of drugs, the emergency use of chemotherapeutics in their free state according to the traditional concept is unavoidable when the drug treatment is urgently needed to save the lives of specific individuals such as humans or animals that have been afflicted with acute infections by pathogenic bacteria, for example. However, where large amounts of drugs is allowed to diffuse into the natural environment for which the main purpose is a preventive effect by the indiscriminate killing of harmful microorganisms estimated to be scanty as well as of a vast majority of environmental microorganisms, the urgent necessity of the drug use and the importance of the accumulative environmental degradation are weighed in balance, and then, it is desirable to cease the use that permits the drugs to diffuse freely.

Further, if in the emergency drug treatment of humans or animals, drugs such as anticancer agents, insecticides, and bactericides that are restricted in their use, or can not be used because of severe adverse reactions through conventional systemic administration methods can be prevented from freely diffusing into their environments, according to this invention and can be used with drug-induced sufferings being suppressed at negligible levels, then novel methods for the utilization of medicines and agrochemical can be provided.

Thus, this invention aims at improving the environmental degradation and adverse reactions.

SUMMARY OF THE INVENTION

In a first aspect of this invention, there is provided:

a biologically active polymer product having:
a polymer substrate; and
a biologically active compound moiety having low molecular weight, the portion being covalently bonded to the polymer substrate and exerting selective biological activity,
wherein the biologically active compound moiety exerts the selective biological activity while being covalently bonded to the polymer substrate.

In the invention, it is preferred that the polymer substrate comprise an organic or inorganic polymer. It is also preferred that a graft chain is linked to the surface of the polymer substrate and the biologically active compound moiety is chemically linked to the graft chain. Further, it is preferred that the biologically active compound moiety is chemically linked to the graft chain through an amido or amine linkage.

In addition, the biologically active compound moiety is preferably a chemotherapeutic, and the chemotherapeutic is preferably an antibiotic.

The antibiotic may be at least one antibiotic selected from a group of beta lactam antibiotics; the antibiotic may be at least one antibiotic selected from a group of benzonaphtacenequinone antibiotics; or the antibiotic may be at least one antibiotic selected from the group consisting of tetracycline antibiotics, chloramphenicol antibiotics, macrolide antibiotics, and aminoglycoside antibiotics.

It is preferred that the biologically active compound moiety have a molecular weight of not more than 5,000. It is more preferred that the biologically active compound moiety have a molecular weight of not more than 2,000.

In a second aspect of this invention, there is provided:

a biologically active polymer product having:
a polymer substrate;
two or more graft chains being linked to the surface of the polymer substrate;
pendant chains being branched from the respective graft chains; and
two or more biologically active compound moietys, the portion being chemically linked to each of the pendant chains and exerting biological activity selectively.

In the invention, it is preferred that the biologically active compound moiety be linked to the pendant chain through an amido or amino linkage.

In a third aspect of this invention, there is provided:

a method for producing a biologically active polymer product having:
a polymer substrate comprising a synthetic, organic polymer material;
graft chains being linked to the surface of the polymer substrate; and
an antibiotic portion being chemically linked to the graft chain, the method having the steps of:
irradiating the polymer substrate comprising a synthetic, organic polymer material with electron beam to provide an active site for graft formation;
exposing a monomer to the polymer substrate to form the two or more graft chains linked to the surface of the polymer substrate; and
allowing an antibiotic to react with the polymer substrate having the graft chains to form the antibiotic portion chemically linked to the graft chain.

In the invention, it is preferred that the antibiotic have a first functional group and the graft chain have a second functional group, wherein the first functional group can react with the second functional group to form an amide group and that the foregoing reaction step be carried out in the presence of a condensing agent. For example, the first functional group is an amino group; and for example, the second functional group is a carboxyl group or a derivative thereof. Alternatively, the first functional group is a carboxyl group or a derivative thereof and the second functional group is an amino group.

It is also preferred that the monomer in a gas-phase state be exposed to the polymer substrate.

In a fourth aspect of this invention, there is provided:

a biologically active polymer product having:
a polymer substrate;
two or more graft chains being linked to the surface of the polymer substrate; and
an antibiotic portion being linked to the graft chain through an amide linkage or an amino linkage, the portion represented by the following formula:

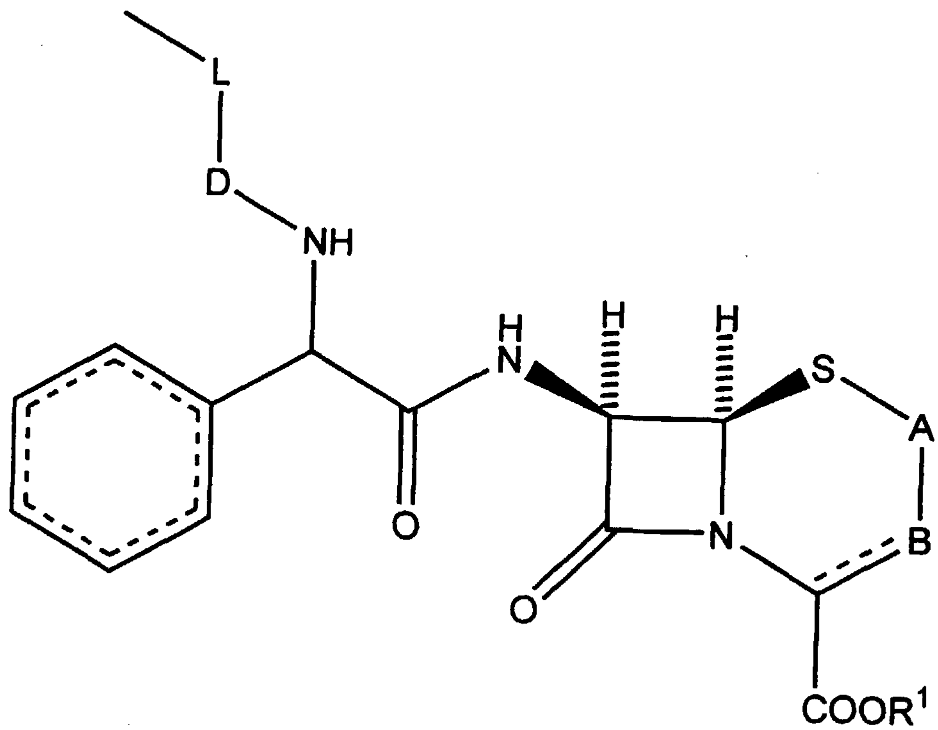

wherein L represents the graft chain portion linked to the surface of the polymer substrate;
$R^1$ is a hydrogen atom, a cation, or a group represented by the following formula:

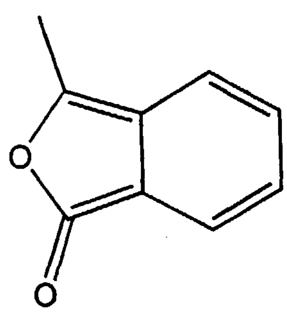

,

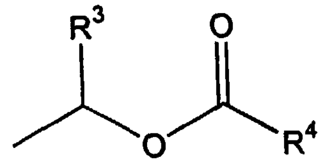

wherein $R^3$ and $R^4$ each independently represents a hydrogen atom, or a straight- or branched-lower alkyl group or a straight- or branched-lower alkoxy group each having 6 or less carbons, with the proviso that $R^3$ and $R^4$, together, may form a saturated or unsaturated 5- to 7-membered ring and a benzene ring may further be fused to the 5- to 7-membered ring,
"A" represents no bond or a methylene group;
"B" is a group represented by the formula: $C(R^5)$, or a group represented by the formula: $C(R^5)_2$, wherein $R^5$ is a lower alkyl group having 6 or less carbons, or a group represented by the formula: $-CH(R^6)O-C(=O)R^7$, wherein $R^6$ is a hydrogen atom or a lower alkyl group having 6 or less carbons and $R^7$ is a lower alkyl group having 6 or less carbons;
"D" represents no bond or a carbonyl group; and
- - - represents a single bond or a double bond.

In the invention, it is preferred that the antibiotic portion be linked to the graft chain through a pendant chain. It is also preferred that the hydrocarbon 6-membered ring in the above formula be a benzene ring and $R^5$ be a methyl group. "D" is, preferably, a carbonyl group. "A" may be no bond or a methylene group.

In the first aspect of this invention, the biologically active substance is linked to the polymer substrate so as not to be released; therefore, it does not happen that any biologically active substance that has been released diffuses into an environment during a period of its use and causes environmental degradation.

In the past, it was widely believed that when a drug was immobilized to a polymer through a covalent bond, the selective biological activity peculiar to the immobilized drug could not be manifested unless the drug was liberated. The present inventors discovered the following: If among possible modes of linking that allow a drug having desired biological activity to be linked to a polymer substrate, a mode of linking is selected based on the double condition: (1) the drug is allowed to be linked to the polymer substrate through such covalent bond that will not cause any liberation of the drug; (2) the drug exerts the biological activity of its own while being linked; and then the drug is immobilized to the polymer substrate, the biological activity peculiar to the drug can be manifested without any drug molecules being liberated.

One example will be explained by way of a preparation obtained by immobilizing ampicillin, which is a kind of beta lactam antibiotic, to a graft polymer, which serves as a polymer substrate. When the amino group of ampicillin was allowed to be linked to a carboxyl group of the substrate side through an amido linkage as is shown in FIG. 1A as attached, the antimicrobial activity peculiar to ampicillin was manifested; whereas, when the carboxyl group of ampicillin is allowed to be linked to an amino group of the substrate through an amido linkage as is shown in FIG. 1B, the immobilized ampicillin displayed no antimicrobial activity at all

Here, the expression "exerts (the) selective biological activity" means that substantially no harm or tolerable adverse reactions are brought to a host environment (nature, a host animal or plant, cells, and organelle, etc.) and that the biological activity is exerted against a harmful, target agent the elimination or the control of which is wanted. Therefore, chemotherapeutics are mentioned as the biologically active compounds exerting their selective biological activity. The chemotherapeutics are drugs having high selective toxicity that causes only damage to harmful agents to be targeted without damaging hosts. The objects of chemotherapeutics are not limited to living things such as pathogenic microorganisms or harmful animals and plants, but extend to a considerably wide range, including cells such as malignant tumors, and non-living matters such as enzymes, receptors, and hormones.

To exert desired selective biological activity peculiar to a drug, the biologically active polymer product of this invention, preferably, not only satisfies the requisite conditions on the mode of linking between drug and substrate, but also has a microenvironment of action or a physical space suitable for the manifestation of effects by the drug immobilized to the substrate. Such physical space that is necessary for drug action can be created by the side chain on the polymer substrate which is generally referred to as a "spacer." The spacer which can be used in the invention preferably has a functional group capable of covalently and stably linking a drug molecule, as well as a chain having the length and properties necessary for manifestation of the selective biological activity peculiar to the drug, while the immobilized drug molecule is as it remains. When a spacer is to be provided, a biologically active compound may be linked to the spacer portion that has previously been linked to a polymer substrate; alternatively, a biologically active compound having previously linked to the spacer may be allowed to be linked to a polymer substrate at the tip of the spacer. However, the spacer is not limited to the graft chain being branched from a polymer substrate, which will be described later, nor is it limiting insofar as it provides the gap between the linked drug molecule and the substrate with an appropriate distance and environmental nature.

In the second, the third, and the fourth aspects, whether or not "the biologically active compound moiety exerts its selective biologically activity while being linked to the polymer substrate" should not be questioned.

## BRIEF DESCRIPTION OF THE DRAWINGS

FIG. 1A shows the state where the amino group at the side chain was used to be immobilized to the carboxyl group of a polymer substrate while the carboxyl group at the 3-position of ampicillin was maintained as such.

FIG. 1B shows the state where the carboxyl group at the 3-position of ampicillin was used to be immobilized to the amino group of a polymer substrate.

FIG. 2 shows the result of evaluation of the antimicrobial activity of ampicillin-immobilized grafted fibers with carboxyl groups against Staphylococcus aureus FDA209P.

FIG. 3 shows the result obtained when the antimicrobial activity of ampicillin-immobilized grafted fibers with carboxyl groups against Staphylococcus aureus FDA209P was evaluated in terms of the relationship between the area of sections of ampicillin-immobilized grafted fibers with carboxyl groups and their antimicrobial activity.

FIG. 4 shows the result of evaluation of the anti-yeast activity of benanomicin A-immobilized grafted fibers with amino groups against Candida albicans 3143.

FIGs. 5A and 5B are the schematic diagrams of an embodiment of biologically active polymer products of this invention.

FIG. 6 shows an ampicillin-like substance immobilized to a polymer substrate.

FIG. 7 shows a beta lactam-like antibiotic immobilized to a polymer substrate.

PREFERRED EMBODIMENTS FOR CARRYING OUT THE INVENTION

(1) Biologically Active Compounds

In carrying out one embodiment of this invention, it is, first, necessary to select a biologically active compound having desired biologically activity against a target. Second, basic information on the chemical structure-activity relationship of the selected biologically active compound is collected, and that such functional group as satisfying the linking prerequisite conditions stated above is present in the drug molecule is determined. If such functional group is not present in the drug molecule, a usable functional group can also be introduced to the drug according to techniques in organic chemistry or in biochemistry, for example.

More specifically, once the target is first decided, a group of candidate drugs that is considered appropriate is selected, for example, from a known drug database, using their biological activity against the target as an index. Then, the most suitable compound can be determined from the group of candidate drugs according to conventional evaluation-testing methods; or unknown drugs having novel pharmacological activity against the target can also be screened.

Secondly, the structure-activity relationship of the selected biologically active compound is investigated; and such functional group or atom at a specified position (position which serves as the active site for immobilization) that the biologically activity peculiar to the drug does not abate even when other compounds are allowed to covalently be linked to the molecule of the selected drug is, preferably, selected. When no functional group available for linking to other compounds is found as the original chemical structure of the biologically active compound remains intact, a new functional group can also be introduced to the drug according to techniques in organic chemistry or in biochemistry.

Among preferable drugs which can be utilized in this invention, those described below can be illustrated as the most important chemotherapeutics: griseofluvin, vernamycin B, ostreogrycin G, isoniazid, benzonaphtacequinones such as benanomicin and pradimicin, pyridoxine, PAS, pimaricin, fungichromin, formycin, toyocamycin, chloramphenicol, tetracycline, streptomycin, erythromycin, ampicillin, norcardicin, SQ 83360, and OA-6129, etc.

Among these chemotherapeutics, preferred are beta lactam antibiotics, benanomicin and pradimicin antibiotics, tetracycline antibiotics, chloramphenicol antibiotics, macrolide antibiotics, and aminoglycoside antibiotics, etc. Ampicillin, cephalexin, cefotaxime and the like can be mentioned as examples of the beta lactam antibiotics. In addition, minocyclin or the like can be mentioned as an example of the tetraccycline antibiotics, chloramphenicol or the like as an example of the chloramphenicol antibiotics, streptomycin or the like as an example of the aminoglycoside antibiotics, erythromycin, leucomycin, oleandomycin or the like as an example of the macrolide antibiotics.

(2) Polymer Substrates (Matrices)

In this invention, the polymer substrate that is used to allow the linking of a biologically active compound exerting selective biological activity (which hereinbelow may be simply referred to as "substrate") is not particularly limited if it is a polymer that does not render the drug diffusive by decomposing or releasing it during the period when the biologically active compound is exerting its biological activity (i.e., during the effective period of product). Therefore, either an organic polymer or an inorganic polymer may be good for the polymer substrate of the invention. Although a variety of substrates can be used, the desirable characteristics with which the substrate should be provided can be, for example, referred to those described in Pure and Appl. Chem. Vol. 67, No. 4, pp. 597-600. Specifically, they are as follows:

i. Insolubility under the use conditions;
ii. Large capacity of immobilization; and
iii. Chemical inertness and strong mechanical strength.

The substrate in this invention may be selected depending on the method for immobilizing the biologically active compound, as well as on the utility of the product.

First, it is necessary to select a substrate suited to the mode and conditions of immobilization reaction and to an environment for its use. For example, a physicochemically stable inorganic or organic polymer must be selected if severe conditions of use such as high temperature, high pressure, strong acidity, and strong alkalinity are anticipated; and a natural polymer may preferably be selected if the treatment of humans or animals is supposed.

Secondly, the selected substrate preferably has a functional group suited to immobilizing the selected biologically active compound stated above.

Furthermore, in order for the biological activity to be exerted while the biologically active compound is immobilized, the provision of a spacer is practically desirable that can create an adequate distance from the substrate and a microenvironment of action.

(2-1) Substrates Comprising Organic Polymers

The organic polymers are preferably synthetic organic polymers, or may be resins. The substrate comprising the organic polymer may easily be processed into a membranous, reticulate, or spherical form.

(2-1-1) Substrates Comprising Organic, Synthetic Polymers

The organic synthetic polymers, which can be mentioned, are, among others, as follows:

synthetic vinyl polymers, including polyolefins such as polyethylene and polypropylene, polyethers such as polyethylene oxide and polypropylene oxide, polyacrylic acids such as polyacrylonitrile, polyacrylates, and polymethacrylates especially,
poly(hydroxyethyl)methacrylates, polyacrylamide, vinyl copolymers such as polystyrene, poly(vinyl acetate), poly(vinyl alcohol), ethylene vinyl acetate copolymer, poly(vinyl acetate styrene); and condensation polymers such as polyesters and nylons.

(2-1-2) Substrates Comprising Other Organic Polymers

Mentioned as other organic polymers are natural organic polymers and modified natural organic polymers, which have been conventionally used to form the substrates for gel filtration, ion-exchange chromatography, affinity chromatography or the like. To specifically name these: cellulose, agarose, glucomannan, chitosan, pullulan, starch, dextran, etc. Conventionally, the substrates for use in affinity chromatography and the like have been processed as porous spheres of these natural organic polymers.
In contrast, mentioned as the materials that can be processed into fibrous or membranous forms are natural, organic polymers such as cellulose, which constitutes cotton fibers, flax fibers or the like, and linear proteins, which constitute wool fibers, silk fibers or the like.

(2-2) Substrates Comprising Inorganic Polymers

Mentioned as an inorganic polymer is silicone resin (also referred to as "silicone"). To activate a polymer comprising linear silicone resin as its backbone, the following method is, for example, mentioned: a silicon compound, which constitutes the principal chain of the silicone resin, is made into hydrosilylated silicon; alternatively, silicone, which has hydroxylated silicon as the constituting unit at branching sites, is used, epichlorohydrin is allowed to act on this, and thus the hydrosilylated portion is activated through epoxidation to form an epoxy-activated silicone.

(2-3) Preferred Processed Forms of Substrate

The synthetic or natural organic polymers, or the inorganic polymers can be shaped and processed into fibrous, membranous or spherical forms depending on the purposes of their use. In addition, those in the form of fiber can be processed into reticulate forms of various meshes for use. Furthermore, the substrate in the form of fiber, membrane, or sphere may be porous.
These substrates may be held by supports, aggregates or the like that comprises other metals or ceramics, or other materials.
The biologically active polymer product of this invention may directly link a biologically active compound through a suitable spacer by utilizing active sites of a polymer or other polymer that comprises the skeleton of the fibrous form substrate, the membranous form substrate or the spherical form substrate; or it may link a low-molecular pharmacological compound by branching the polymer that comprises the skeleton of the substrate into graft chains and by utilizing the active sites (functional groups for linking) provided on those graft chains.

(2-4) Graft Formation of Polymer Substrates

In immobilizing a biologically active compound to the polymer substrate, it is preferred that graft chains having suitable functional groups (e.g., carboxylic acid or amino group) be linked to the polymer substrate to effect graft polymerization.
The method of providing active sites for linking graft chains to the polymer substrate can utilize a method that is generally used, such as treatment by irradiation with ionized radiation or oxidation treatment with one or more kinds of oxidants. Especially preferred is a method of irradiation with ionized radiation using electron beam or cobalt 60 as a ray source. When the substrate is synthesized by vinyl polymerization, there may be a method in which a copolymerization monomer is mixed to cause copolymerization and to provide active sites comprising vinyl groups at the side chains of the substrate polymer. In addition, a liquid-phase graft polymerization method, a gas-phase graft polymerization method or the like can be employed as the graft polymerization method; and generally preferred is the gas-phase graft polymerization method that makes the monomer into its gas-phase state and contacts it with the substrate.

In the graft formation, graft chains formed therefrom can be provided with the role as the spacer. Since a biologically active compound is linked to the terminus of, or along the graft chain, the biologically active compound molecule that is linked is to be disposed at a position away from the substrate, and the biologically active compound can have an interaction space and a microenvironment that are requisite for manifestation of the biological activity peculiar to the drug. Particularly, if the graft chain is sufficiently elongated so as to be able to move according as the conditions such as the flow of fluid or the concentration gradients of substance, the probability of contact between the immobilized drug molecule and the target can be enhanced. Further, the biologically active compound links not only to the terminus of a graft chain, but also to a pendant along the graft chain; therefore, one graft chain will normally be linked to a plurality of biologically active compounds and through the optimization of arrangement of the graft chains, as well as the number of pendants, the utilization efficiency of the drug can be enhanced.

The functional group that is provided along or at the terminus of the graft chain arranged on the polymer substrate to immobilize a biologically active compound is optimized depending on the type and mode of the functional group on the biologically active compound molecule. For example, if a carboxyl group (or an amino group) is provided at the graft side chain, a stable peptide linkage can be constructed by the carbodiimide method using an amino group (or a carboxyl group) of the biologically active compound.

(3) Types and Modes of Linkages for Use in the Immobilization of Biologically Active Compounds

As already stated, the linkages that can be used for the immobilization of biologically active compounds in this invention must be strong covalent bonds so that liberation of the biologically active compounds might not occur in their use environments. Although the definition of conventional immobilization embraces linking techniques such as embedding, ionic linking, milling, and nonspecific adsorption, naturally they can not be used in the invention supposing that liberation of the biologically active compounds into the environment would not occur. Therefore, the linking between the biologically active compound and the polymer substrate employ a covalent bond in the invention.

A spacer that is necessary for linking the biologically active compound to the polymer substrate has been provided on the substrate side, and then, the biologically active compound can be immobilized to the terminus of the spacer. Alternatively, a spacer has been attached to a selected active site of the biologically active compound, and then, the low-molecular pharmacological compound having the spacer can be immobilized to the "substrate having an active site" through the spacer.

The main portion of a suitable spacer that can be utilized in this invention may be any chain form insofar as it can link "the active site provided on the substrate" or "the active site provided at the graft chain branched from the substrate" with "the functional group of the drug," and in addition, can effectively be used to give a microenvironment suited to the manifestation of action of the drug. Specifically, a straight methylene ($-CH_2-$) chain, a straight oxyethylene ($-O-CH_2-CH_2-$) chain, or the like can advantageously be used, but it needs to be a linking group that would not cleave even under severe conditions such as high temperature, acidity, alkalinity, or the like.

The functional groups that can be used at "the active site provided on the substrate," or at "the active site provided at the graft chain branched from the substrate," or at "both termini of the spacer (may not be the same groups)," or at "the specified position of the biologically active compound for linking" and that can be mentioned are, for example, as follows:

a double bond, a triple bond, an amino group, an epoxy group, a glycyl group, an isocyanate group, an aldehyde group, a carboxyl group or a derivative thereof (e.g., an ester group, an anhydrous carboxylic acid group and a halogenated carbonyl group), an allyl group, etc.

In addition, as the active site in an inorganic polymer substrate, a hydrosilyl group in silicone resin can be mentioned.

The active site provided on the substrate, the active site provided on the graft chain branched from the substrate, both termini of the spacer, or the functional group in a low-molecular pharmacological compound is an amino group and the low-molecular pharmacological compound having a carboxyl group as the activating group is to be immobilized; in such a case immobilization can be conducted using a carbodiimide condensation agent such as 1-ethyl-3-(3-dimethylaminopropyl)carbodiimide.

Further, the active site provided on the substrate, the active site provided on the graft chain branched from the substrate, both termini of the spacer, or the functional group in a low-molecular pharmacological compound is a hydroxyl group and the low-molecular pharmacological compound having an amino group as the activating group is to

be immobilized; in such a case the terminal hydroxyl group is converted to a terminal aldehyde group by periodic acid oxidation, a Schiff base bond is then formed by the action of the low-molecular pharmacological compound having a terminal amino group, and the Schiff base bond is converted to a chemically stable carbon-nitrogen linkage ($-CH_2-NH-$) by being reduced, which can thus effect linking.

Furthermore, in this invention there are some linkages that may not be used because of steric hindrance or unsuitability of the microenvironment where the drug manifests its biological activity in the product, depending on the kind and the linking mode of the low-molecular pharmacological compound. Therefore, it is necessary to determine the presence or absence of manifestation of any biological activity with respect to individual pharmacological compounds and substrates.

In this invention, where a penicillin or cephalosphorin derivative having an amino group at its side chain and a carboxyl group on its mother neucleus such as ampicillin, cephalexin, or cefotaxime is allowed to be linked to the substrate, it has been ascertained that the drug exert its efficacy when the amino groups at the side chain is covalently bonded to the carboxyl group of the substrate while the carboxyl group of the penicillin or cephalosporin skeleton is maintained as such.

In FIG. 1A, the amino group at the side chain of ampicillin is linked to the carboxyl group of the substrate. In this instance, the linked ampicillin exerts its efficacy because the carboxyl group on its mother neucleus is maintained as such. In contrast, the carboxyl group on the mother neucleus of ampicillin is linked to the amino group of the substrate in FIG. 1B. In this instance, the immobilized ampicillin does not exert any efficacy.

FIG. 6 shows the ampicillin-like substances immobilized to the polymer substrate. The immobilized ampicillin-like substances, as shown in FIG. 6, also exert selective biological activity. In FIG. 6, "L" shows a spacer linking to the polymer substrate. $R^1$ is a hydrogen atom, a cation, or a group as shown in the following:

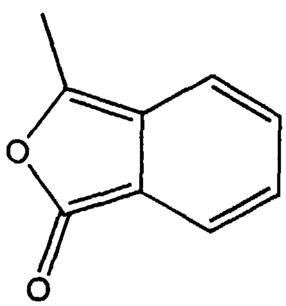

,

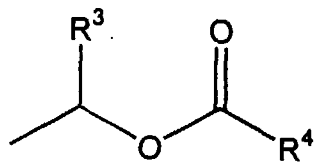

In the formula, $R^3$ and $R^4$ each independently represents a hydrogen atom, or a straight- or branched-lower alkyl group or a straight- or branched-lower alkoxy group each having 6 or less carbon atoms, with the proviso that $R^3$ and $R^4$, together, may form a saturated or unsaturated 5- to 7-membered ring and a benzene ring may further be fused to the 5- to 7-membered ring. $R^2$ represents a hydrogen atom.

When $R^1$ is a cation, it represents a carboxylate. The cation is not particularly limited, and may be either an inorganic cation or an organic cation. Mentioned as the inorganic cation are an alkaline metal cation such as sodium ion or potassium ion, an alkaline earth metal ion such as calcium ion, a transition metal ion such as ferric ion (or ferrous ion), a complex ion, etc. Mentioned as the organic ion is, for example, an ammonium ion such as a tetraalkylammonium ion.

Cephalexin and like substances thereof are shown below:

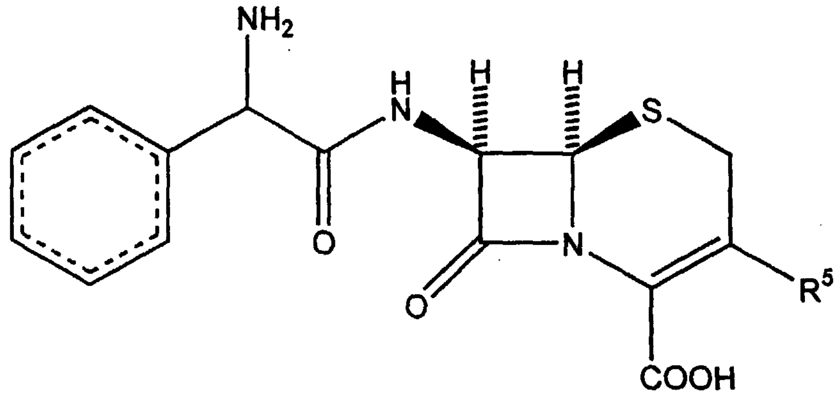

In the formula, $R^5$ is a lower alkyl group having 6 or less carbons such as a methyl or ethyl group, or a group represented by the formula: -CH($R^6$)O-C(=O)$R^7$, wherein $R^6$ is a hydrogen atom or a lower alkyl group having 6 or less carbons. $R^7$ is a lower alkyl group having 6 or less carbons.

- - - represents a single or double bond.

A benzene ring and cyclohexane-1,4-diene are preferable as the hydrocarbon 6-membered ring in the formula.

Mentioned as the compounds represented by the above formula are, for example, cephaloglycin ($R^5$ is a group represented by $-CH_2O-C(=O)CH_3$ and the hydrocarbon 6-membered ring is a benzene ring), cephalexin ($R^5$ is a methyl group and the hydrocarbon 6-membered ring is a benzene ring), and cepharadine ($R^5$ is a methyl group and the hydrocarbon 6-membered ring is cyclohexane-1,4-diene).

In consideration of the ampicillin-like and cephalexin-like substances, the beta lactam antibiotics immobilized to the polymer substrate as shown in FIG. 7 are also thought to exert the selective biological activity.

In FIG. 7, "L" represents the graft chain portion linked to the surface of the polymer substrate;

$R^1$ is as defined above.
"A" represents no bond or a methylene group.
"B" is a group represented by the formula: C($R^5$), or a group represented by the formula: C($R^5$)$_2$, wherein $R^5$ is as defined above.
"D" represents no bond or a carbonyl group (-C(=O)-).
- - - represents a single or double bond.

A benzene ring and cyclohexane-1,4-diene are preferable as the hydrocarbon 6-membered ring in the formula.

In this invention, the antibiotic substance portion may be linked to the spacer such as a graft chain through an amido linkage (-NH-CO-), or the antibiotic substance portion may be linked to the spacer such as a graft chain through an amino linkage (-NH-).

One aspect of this invention resides in secure linking through a covalent bond at the site that would not inhibit the activity of a biologically active compound.

A preferred embodiment of linking the biologically active compound to the substrate in this invention is as follows: in carrying out graft polymerization, the graft polymerization is conducted on the substrate provided with an active site to allow a graft chain to link to the substrate using a monomer, according to the method that will be explained below.

In conventional graft polymerization methods, the liquid-phase graft polymerisation method that allows direct contact between substrate and monomer was popular. However, since large amounts of monomers and washing chemicals are needed, there is a disadvantage that the running cost becomes high. Especially, in the case of a porous substrate larger amounts are needed and besides, the time required for cleaning is lengthy. In contrast, the gas-phase graft polymerization method, by which a monomer is converted to its gaseous state and then it is allowed to contact with the substrate, needs only a very small amount of monomer and no washing; and the method is advantageous from the standpoint of time and cost, although the airtightness of a polymerization device requires attention. Further, the problem of ununiformity in polymerization that will become a concern in the gas-phase graft polymerization has disappeared due to an improvement on the polymerization device.

In this invention, the polymer substrate that will form grafts may be either an organic polymer or an inorganic

polymer.

The functional groups as already described in the section of "Types and Modes of Linkages for Use in the Immobilization of Biologically Active compounds," among others, may be mentioned as the functional group for immobilizing a biologically active compound that is provided in or at the terminus of the graft chain provided on a polymer substrate. For example, when a carboxyl group (or an amino group) has been provided at the grafted side chain, this terminal group and the amino group (or the carboxyl group) of the biologically active compound can be linked, according to the carbodiimide method.

The concrete methods for providing an active site such as a carboxyl group or an amino group in or at the terminus of the graft chain provided on the polymer substrate, which can be mentioned, are, for example, as follows:

(i) a method for graft polymerization using acrylic acid or methacrylic acid as a graft monomer or a graft copolymerization monomer (the functional group being a carboxyl group);
(ii) a method for graft polymerization using amino styrene as a graft monomer or a graft copolymerization monomer (the functional group being an amino group); and
(iii) a method for graft-polymerizing vinyl acetate with its use as a graft monomer or a graft copolymerization monomer, thereafter utilizing the hydroxyl group of vinyl alcohol formed as the result of the saponification of a vinyl acetate unit and substituting it with a spacer that has an epoxy group and an amino group at its termini (the functional group being an amino group).

Nevertheless, the method for providing an active site in the graft chain or at its terminus is not limited to those mentioned: they are adequately optimized depending on the chemical characteristics peculiar to selected biologically active compounds.

Further, in this invention the immobilization of a biologically active compound has a purpose of preventing the biologically active compound from freely moving and diffusing within its environment. Therefore, when the biologically active compound is immobilized to the spherical substrate described above, it is preferred that the immobilization be conducted so that the spherical substrate may not disperse into the treatment space (e.g., a filtration bed provided on the passageway for filtering the air containing treatment objects) of objects to be treated (bacteria, molds, etc).

FIGS. 5A and 5B are the schematic diagrams of biologically active polymer products of one embodiment of the invention. The biologically active polymer product has a polymer substrate 10 and two or more graft chains 12 linked to the surface of the polymer substrate 10. A pendant chain 14 is branched from each of the graft chains 12. The pendant 14 may be branched from along the graft chain 12, or may be branched from its terminus. Further, in one aspect of the invention, two or more biologically active compound moietys B are covalently bonded to the respective pendant chains 14. The biologically active compound moiety may be directly linked to the pendant chain 14, or may be linked through a spacer. This enables one graft chain to support two or more biologically active compounds, which can enhance the density of the biologically active compound on the polymer substrate.

Used as the graft 12 is, for example, a copolymer of an olefin, such as ethylene or propylene, and a monomer having a functional group such as acrylic acid. Thus, the pendant 14 can be formed, for example, by polymerizing the monomer from the acrylic portion of this copolymer. When the monomer having a functional group such as acrylic acid is used in forming the pendant 14, a carboxyl group or a derivative thereof that reacts with the biologically active compound moiety B can be introduced.

(4) Assays for Biological Activity

Conventionally, the following quantitative evaluation method has been adopted in the case of evaluation of the biological activities of drugs: assuming that a drug is dissolved in solution, the magnitude of its efficacy against a target (a test agent) is determined by the dilution method or the diffusion method, and the presence or absence of efficacy of the drug is evaluated based on its potency. However, since the biologically active compounds can not freely disperse with respect to the products of this invention, the test evaluation system prescribed by the Drugs, Cosmetics and Medical Instruments Act is not applicable.

In this invention, therefore, test conditions where a target is forcedly allowed to contact a test drug-immobilized polymer substrate are set, and the magnitude of its efficacy is quantitatively evaluated. Namely, the present inventors tested the functions of products according to a rapid evaluation method for antibiotic activities which will be explained below. First, to 5 ml of liquid medium are added one or two drops of a preliminary culture solution of a test microorganism that has been shake-cultured from the middle period of its logarithmic growth phase up till a late period thereof, and a provided polymer product (section of 1.0 x 1.0 cm in the case of a product in the form of membrane). It is shake-cultured at a temperature suited to the growth of the test microorganism. For example, when Bacillus subtilis ATCC6633, Staphylococcus aureus FDA 209P, Escherichia coli NIHJ or the like is to be used as the test microorganism, a Nutrient Broth medium can be used as medium; and the culturing temperature of 30°C and the overnight culturing time prior to

shaking can be employed.

A polymer substrate (1.0 x 1.0 cm) that supports a biologically active compound to be a test subject is placed in a culture medium, 5 ml; one or two drops of an overnight-cultured solution containing a test microorganism is inoculated in it; absorbance is measured at 600 nm using a spectrophotometer; and the growth curve of the test microorganism is determined over 6 - 8 h. At the same time, carrying out similar manipulations without addition of the provided polymer product, the normal growth curve of the test microorganism is determined, which serves as an ineffective control.

Usually, the test microorganism culture solution reaches the logarithmic growth phase or the stationary phase after culturing for 6 - 8 h, which is the measuring time. At this point, if similar manipulations are carried out in parallel on a polymer substrate linking to no drug, the activity of the drug linked to the polymer substrate can be evaluated more accurately.

In the rapid evaluation method for antibiotic activities, when the provided polymer product is in the form of particles, about 0.25 ml of the provided polymer product is used in place of a section of 1.0 x 1.0 $cm^2$ and similar manipulations are carried out. In addition, even when the provided polymer product is in the form other than membrane and particles, it can be evaluated according to a method similar to those for polymer products in the form of membrane or particles. In this instance, if the polymer product is dispersed within a medium and the growth of a test microorganism can not be monitored by the absorbance at 600 nm, said culture solution is sampled at suitable intervals to be observed under an optical microscope and the activity can be evaluated by comparing it with a test microorganism culture solution which is the control.

The test microorganisms for use in this invention can employ microorganism cell strains that are used in ordinary evaluation of antibiotic activities, as well as standard cell strains preserved in various cell-preserving institutions and microorganisms isolated from nature. For example, the following can be mentioned: Staphylococcus aureus FDA209P; Staphylococcus aureus Smith; Staphylococcus aureus K2; Bacillus subtilis ATCC6623; Bacillus anthracis; Mycobacterium 607; Mycobacterium ATCC15483; Mycrococcus luteus ATCC9341; Proteus vulgaris OX-19; Klebsiella pneumoniae PCI 602; Escherichia coli NIHJ; Escherichia coli JM109; Escherichia coli MV1190; Pseudomonas aeruginosa; Salmonella enteritidis; Candida albicans 3143; and Trichophyton mentagrophytes.

Further, in the rapid evaluation method for antibiotic activities, it is preferred that with respect to selected test microorganisms, their media, culturing temperatures, and culturing times prior to shaking, which are optimum for their growth, be set.

Under the present circumstances, it is impossible to quantitatively evaluate the amounts of drugs immobilized to substrates, either chemically or biologically, with the accuracy that is comparable to test methods based on the diffusion method applicable to free drugs. Furthermore, as for the substrates to which drugs are immobilized, there exist no established theories that are scientifically accepted as far as methods for the quantitative evaluation of their activity as antibiotics are concerned.

In the test methods based on conventional diffusion methods applicable to free drugs, the prerequisite is that media, glass apparatuses and the like are sterilized, besides, the drugs themselves that are the subjects of evaluation are manufactured and stored aseptically. It is, therefore, possible to evaluate the effects of the drugs on the growth of selected test microorganisms accurately. In contrast, among polymer substrates that are the subject of this invention, there are some of such nature that they can not be sterilized in an autoclave at 121°C. The rapid evaluation method for antibiotic activities of the invention, therefore, aims at the evaluation under the conditions where even if the method is subject to influence by microorganisms other than the test microorganism present in an environment such as air, the influence can be eliminated or ignored.

The rapid evaluation method for antibiotic activities is an evaluation method similar to the shake flask method: it is adopted by the Fabric Product Hygienic Processing Council when the Council issues certification marks (SEK marks) to antimicrobial deodorizing processed fabric products, and mainly, it is an evaluation method for the effectiveness of products that are treated with processing chemicals of the non-elution type. (Techniques in the Use of Antimicrobial and Anti-Mold Agents, and the Testing and Evaluation of Antimicrobial Potency 244; the Technical Information Association Ed.; the Technical Information Association; 1996.) According to the shake flask method, a provided fabric product that is cut in about 1.0x1.0 cm and a provided microorganism culture solution are added to a flask to which phosphate buffer solution has been added and which is then sterilized in an autoclave at 121°C. After the body cells are allowed to contact the provided fabric product by shaking for 1 h, the number of viable cells is counted by the mixed dilution argar plain plate method, and the viable cell number is to be compared to the control that has been treated similarly without addition of the provided fabric product.

In the test method based on conventional diffusion methods applicable to free drugs, the time of contact between the test microorganism and the drug is maintained for 24 h or longer, and the effect on the microorganism is evaluated. In contrast, the condition that is common to the rapid evaluation method for antibiotic activities of this invention and the shake flask method is that the time of contact between the test microorganism and the drug is set to be brief. The reason is the following: Even where microorganisms other than the test microorganism are present in the culture solution at the start of testing, if their presence is very small when compared to the amount of addition of the test microorganism, it is thought that the influence of the microorganisms other than the test microorganism can be eliminated or ignored under this condition as long as the evaluation is conducted within the culturing time of 6 - 8 h during which an ordinary test microorganism reaches the latter period of its logarithmic growth phase or its stationary phase.

Additionally, in the rapid evaluation method for antibiotic activities, by using a spectrophotometer while comparing with the control that has no efficacy at all times, the absorbance of the test microorganism culture solution at 600 nm-wavelength is measured in a time-dependent manner and the growth process of the test microorganism is monitored. Thus, the influence of microorganisms other than the test microorganism can be eliminated or ignored, and it can be said that the method is a reasonable evaluation method under the present circumstances.

(5) Examples of Utilization of Biologically Active Polymer Products of the Invention

In this invention, a biologically active compound having selective biological activity is covalently bonded to a polymer substrate so as not to be liberated, thus making the biologically active polymer product. Accordingly, the characteristics that the biologically active compound linked to the polymer product is not liberated from the polymer substrate mean the following:

(i) No liberated biologically active compound remains in an environment that has been treated with the biologically active polymer product of the invention; and
(ii) It is required that the biologically active polymer product be contacted directly with a target.

According to the first example of utilization, when fluids such as blood, body fluid, water, drinks, air, foods, and feeds are forcedly conveyed and briefly contacted with the biologically active polymer products of this invention, targets can be removed from the fluids without the fear that liberated drugs might remain in the fluids after treatment and might bring undesirable influence. For such utility, products of the invention such as those in the form of membrane, a net, or a spherical, filter medium may be used advantageously. In this case the targets that can be mentioned are, for example, a wide range of agents including living things such as viruses, microorganisms, pollen, eggs, insects, and small animals, besides non-living matters such as enzymes, hormones, and toxic substances.

Another example of utilization of the biologically active polymer products of this invention is use forms that suppose the use for considerably long hours. For example, the polymer products of the invention that have bees processed into sheet forms, paint forms, or fibrous forms may be utilized as clothes, curtains, sheets, paint products, external films, sanitary goods, etc.

Thus, a variety of utilization is possible for the polymer products of this invention to which biologically active compounds having selective biological activities are linked, and the examples of their utilization are not to be limited to those illustrated herein.

The concrete utility of this invention is not limited to the fields of medical treatment or the manufacture in agriculture and fishery, and it extends more widely to preservation of the environment concerning, among others, daily life, breeding, and cultivation or water environments (including the preservation of water quality, the prevention of abnormal growth of microorganisms, and the like).

EXAMPLES

The examples of the polymer products of this invention will be illustrated below. Nevertheless, the invention is not to be limited by the examples that follow.

Example 1 Grafted fibers with carboxyl groups Having Immobilized Ampicillin

A nonwoven fabric having a METSUKE of 50 $g/m^2$ and a thickness of 0.4 mm, which was made of polypropylene (available from Mitsui Petrochemical Ind. Co.; the trade name: Syntex PS-110), was irradiated 200 kGy with electron beam (at 2 Mev and 1 mA) under a nitrogen atmosphere. Then, these fibers were immersed in a monomer solution of acrylic acid/methanol (1/9) and were allowed to react at 40°C for 4 h. Consequently, fibers having an acrylic acid graft rate of 78.5% were produced, which are hereinafter referred to as the "grafted fibers with carboxyl groups." The ion-exchange capacity of the grafted fibers with carboxyl groups was 6 meq/g. Here, the graft rate was calculated according to the following formula:

$$\text{graft rate}=\{(\text{weight of substrate after graft formation}-\text{weight of substrate before graft formation})/\text{weight of substrate before graft formation}\}\times 100(\%)$$

The sodium salt of ampicillin (molecular weight of 349.42), 51.3 mg (equivalent to 48.2 mg as converted based on ampicillin free base), 40 pieces of grafted fibers with carboxyl groups that were cut into squares in 1.0 x 1.0 cm (total weight of 513 mg), 1-ethyl-3-(3-dimethylaminopropyl)carbodiimide hydrochloride (water-soluble carbodiimide), 252. 4 mg, were added to 50 ml of a M/100 potassium phosphate buffer (pH 4.4) in a 250-ml Erlenmeyer flask. Shaking was conducted at 5°C for 20 h (revolution radius: 12 cm, the number of revolution: 80 times/min), and ampicillin was immobilized on the grafted fibers with carboxyl groups .

As a control, a linking reaction containing no water-soluble carbodiimide (the grafted fibers with carboxyl groups, 496 mg, and ampicillin hydrochloride, 51.3 mg, were added to 50 ml of a M/100 potassium phosphate buffer (pH 4.4)) was carried out under the same conditions.

After the reaction was over, fibers were taken out and washed with 100 ml of purified water twice. Further, they were washed under shaking in 100 ml of purified water at room temperature for 3 h. To prevent contamination by various bacteria such as mold, an ampicillin-immobilized sample and a control sample that had been already washed were immersed in pure methanol immediately after washing and stored in a refrigerator.

Elemental analysis values of sulfur (S) and nitrogen (N) for these ampicillin-immobilized grafted fibers with carboxyl groups and the control grafted fibers with carboxyl groups are as follows:

ampicillin-immobilized sample: S, 0.131%; N, 1.19%
control sample of grafted fibers with carboxyl groups : S, 0.00007%; N, 0.02%

Since S is derived from ampicillin, on the basis of the S elemental analysis values it is calculated that in this immobilization experiment a total amount of 7.41 mg of ampicillin was immobilized to 513 mg of the grafted fibers with carboxyl groups. Thus, it has been proved that 185 μg per piece of ampicillin is immobilized in a piece of the ampicillin-immobilized grafted fibers with carboxyl groups. Although the elemental analysis values for N are markedly high when compared to those for S, it has been deemed from previous findings that carboxyl groups which are maintained in the carbodiimde-activated state without being utilized in the immobilization of ampicillin coexist in large amounts.

FIG. 1A shows the linking position and the mode of linking between ampicillin and grafted fibers with carboxyl groups. On the other hand, the one (FIG. 1B) in which the carboxyl group of ampicillin was linked to the amino group of grafted fibers with amino groups could not exert the biological activity that was characteristic to ampicillin.

FIG. 2 shows the results from a test of antimicrobial activity of these ampicillin-immobilized sample and control sample against Staphylococcus aureus FDA 209P, as assayed by the rapid evaluation method for antibiotic activities. As is apparent from FIG. 2, the sample having immobilized ampicillin in the presence of carbodiimide displays distinct antimicrobial activity, whereas the sample having undergone similar treatment in the absence of carbodiimide displays no antimicrobial activity at all. This demonstrates that non-specific adsorption of ampicillin to the substrate has not occurred under the immobilization and aftertreatment conditions used here. Further, under similar testing and evaluation conditions, this ampicillin-immobilized grafted fibers with carboxyl groups displayed antimicrobial activity against Bacillus subtilis ATCC6623, Bacillus anthracis, Staphylococcus aureus K2, Staphylococcus aureus Smith, and Mycrococcus luteus ATCC9341, etc. However, even this ampicillin-immobilized sample was ineffective against Escherichia coli NIHJ, Proteus vulgaris OX-19, Klebsiella pneumoniae PCI602, Candida albicans 3143, Aspergillus niger, etc. Namely, it can be said that the ampicillin-immobilized grafted fibers with carboxyl groups according to this invention exert the selective biological activity that is peculiar to ampicillin.

Table 1

| Antimicrobial Spectrum of Ampicillin | |
|---|---|
| bacterium species | final inhibition concentration (μg/ml) |
| Staphylococcus aureus FDA209P | 0.015 |
| Bacillus anthracis | 0.031 |
| Klebsiella pneumoniae | 5.0 |

Even when this ampicillin-immobilized grafted fibers with carboxyl groups was thoroughly washed with high purity deionized water until TOC (total concentration of organisms) of the washed solution reached about 20 μg/l which was equal to the blank value, it maintained antimicrobial activity at the same level as before thorough washing. It continued to maintain antimicrobial activity against Staphylococcus aureus FDA209P even after it was subject to heat treatment at 0°C for 10 min.

FIG. 3 results from an investigation of the correlation between the surface area of a sample immobilized product of ampicillin-immobilized grafted fibers with carboxyl groups and its antimicrobial activity. As is apparent from FIG. 3, the surface area of the sample of ampicillin-immobilized grafted fibers with carboxyl groups (i.e., the absolute amount of ampicillin on a piece of test sheet) has a positive correlation with the antimicrobial activity.

Treatment with 1 M glycine, pH 8.5, and ethanol amine was conducted on these samples at room temperature for 3 h according to a standard method and unreacted carboxyl groups in the activated state were inactivated, but their antimicrobial activity did not change before or after the treatment.

Example 2 Grafted fibers with carboxyl groups Having Immobilized Cephalexin or Cefotaxime

Substituting the sodium salt of ampicillin in the reaction mixture composition according to Example 1 with an equivalent amount of cephalexin or cefotaxime, it was immobilized to the grafted fibers with carboxyl groups under the same conditions. When the antimicrobial activity test was conducted using the rapid evaluation method for antibiotic activities, the cephalexin-immobilized grafted fibers with carboxyl groups as well as the cefotaxime-immobilized grafted fibers with carboxyl groups displayed distinct antimicrobial activity against Staphylococcus aureus FDA209P.

The structural formula for the sodium salt of cefotaxime is shown below:

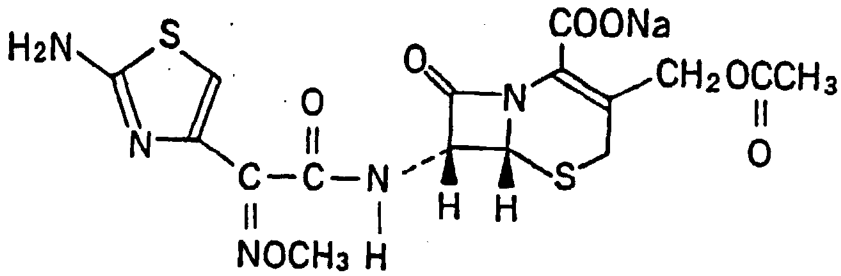

Example 3 Ampicillin-Immobilized Agarose Beads of the Carboxylic Acid Type

A modified natural polymer substrate in the form of beads that used agarose as a base material and that had carboxyl groups as the functional group (the commercial name: ECH-Sepharose 4B), 0.5 ml, was suspended in purified water at room temperature and washed. Then, it was suspended in a M/100 potassium phosphate buffer (pH 4.5), 5 ml. To this were added the sodium salt of ampicillin, 5 mg, and water-soluble carbodiimide hydrochloride, 25 mg, and it was gently stirred at 5°C for 6 h. After the immobilization reaction was over, the ampicillin-immobilized ECH-Sepharose 4B, 0.1 ml, that had been sufficiently washed with purified water was used and its antimicrobial activity against Staphylococcus aureus FDA209P was investigated according to the rapid evaluation method for antibiotic activities, when distinct antimicrobial activity was confirmed.

Example 4 Ampicillin-Immobilized Synthetic Beads of the Carboxylic Acid Type

A porous synthetic resin of the carboxylic acid type in the form of beads (the commercial name: Daiaion WK10) was used as the polymer substrate. After this polymer substrate, 0.5 ml, was suspended in purified water at room temperature and washed, it was suspended in a M/100 potassium phosphate buffer (pH 4.5), 5 ml. To this were added the sodium salt of ampicillin, 5 mg, and water-soluble carbodiimide hydrochloride, 25 mg, and it was stirred at 5°C for 24 h. After the immobilization reaction was over, the ampicillin-immobilized Daiaion WK10 (0.1 ml) that had been sufficiently washed with purified water was used and its antimicrobial activity against Staphylococcus aureus FDA209P was investigated according to the rapid evaluation method for antibiotic activities, which confirmed the manifestation of the antimicrobial activity.

Example 5 Oxirane • Acrylic Acid Beads Having Immobilized Ampicillin

A polymer substrate that had a principal structure comprising methacryl amide and contained epoxy groups at the terminus of its side chains, and that had bead forms (the commercial name: Oypergid), 0.5 g, was suspended in purified water at room temperature and washed. Then, it was suspended in a M/100 sodium potassium phosphate buffer (pH 8.5), 30 ml. To this were added the sodium salt of ampicillin, 100 mg, and it was stirred at 25°C for 24 h.

Through this reaction, the primary amino group (-$NH_2$) of the sodium salt of ampicillin reacted with a terminal epoxy group of the side chain of the polymer substrate to form a secondary amino group (X-NH-$CH_2$-CH(OH)-L). Here, "X" represents an ampicillin portion and "L" represents a side chain portion of the polymer substrate in the formula. Thus, the ampicillin portion which is a biologically active compound moiety is linked to the polymer substrate through an amino linkage.

After the reaction was over, unreacted epoxy groups were inactivated with 8 g glycin/100 ml 0.5 M phosphate buffer (pH 8) according to the instructions by the manufacturer of bead products. Ampicillin-immobilized Oypergid, 0.05 g, was taken, and after washing with purified water, its antimicrobial activity against Staphylococcus aureus FDA209P was investigated according to the rapid evaluation method for antibiotic activities, which confirmed the manifestation of the antimicrobial activity.

Example 6 Cellulose Beads of the Carboxylic Acid Type Having Immobilized Ampicillin or Cephalexin

A modified natural polymer in the form of beads that used cellulose as the base material and that had carboxyl groups as the functional group (the commercial name: CM Cellulofine C-200), each 5 ml, was suspended in purified water at room temperature and washed. Then, it was mixed with each 20 ml of a M/100 phosphate buffer (pH 4.5) and was subject to shaking at room temperature for 30 min. To each was added 250 mg of water-soluble carbodiimide hydrochloride, and it was stirred at room temperature for 30 min. Then, the sodium salt of ampicillin or cephalexin, 10 mg, was added and its immobilization treatment was conducted at room temperature for 5 h. After these beta lactam-immobilized cellulose beads of the carboxylic acid type were sufficiently washed with purified water according to the standard method, their antimicrobial activity against Staphylococcus aureus FDA209P was investigated according to the rapid evaluation method for antibiotic activities, when they displayed strong antimicrobial activity against Staphylococcus aureus FDA209P.

Example 7 Grafted fibers with carboxyl groups Having Immobilized Ampicillin or Cephalexin by Use of 1,1-Carbonylbis-1H-(Carbodiimide)Imidazole (Water-Insoluble Carbodiimide)

10 pieces of the grafted fibers with carboxyl groups (1.0 x 1.0 cm), which were used in Example 1, were immersed in a M/10 phosphate buffer (pH 4.5), they were dehydrated and dried. These sections were placed in anhydrous dimethyl sulfoxide, 5 ml, and the sodium salt of ampicillin or cephalexin, 10 mg, was added, and it was immobilized under anhydrous state at 5°C overnight. After the immobilization reaction, beta lactam-immobilized fiber sections of the carboxylic acid type were sufficiently washed with dimethyl sulfoxide. Similarly to Example 1, their antimicrobial activity against Staphylococcus aureus FDA209P was investigated according to the rapid evaluation method for antibiotic activities, which confirmed the manifestation of the antimicrobial activity.

Example 8 Preparations Having Immobilized Ampicillin by Use of the Substrates Already Activated with Carbodiimide

Two types of commercially available substrate products that had previously been activated with carbodiimide (REACTIGEL (6X) and REACTI-GEL (HW-65F); Products from Pierce Inc.) were used to carry out the immobilization of ampicillin.

After each 5 ml of the substrates was washed with 500 ml of purified water on a glass filter, it was suspended in a M/10 boric acid buffer (pH 9.0), 25 ml, and the sodium salt of ampicillin, 10 mg, was added, which was shaken at room temperature overnight. Ampicillin was thus immobilized on the substrates. After the reaction was over, ampicillin-immobilized substrates were filtrated, washed with water, and they were shaken in an M/10 trisbuffer (pH 8.0), 25 ml, for 15 min. Activated carboxyl groups remaining in the substrates as being unreacted were thus inactivated. To remove ampicillin that might remain in or might be nonspecifically adsorbed to the ineffective voids of the substrates, the substrates were washed with sufficient amounts of water and M/100 phosphate buffer (pH 6.8). Heat shock was repeated on the substrates three times to make sure: the treatment involved maintaining the substrates at 0°C for 5 min, thereafter bringing them suddenly to 100°C to maintain for 5 min, and again bringing them suddenly to 0°C.

As for these thoroughly washed ampicillin-immobilized preparations, when their antimicrobial activity against Staphylococcus aureus FDA209P was investigated according to the rapid evaluation method for antibiotic activities, only the ampicillin immobilized to REACTI-GEL (HW-65F) manifested antimicrobial activity, whereas the sample immobilized to REACTI-GEL (X6) displayed only a negligible level of antimicrobial activity.

Example 9 Grafted fibers with amino groups Having Immobilized Benanomicin A

After electron beam irradiation similar to Example 1 was conducted on the same polypropylene nonwoven fabric as that of Example 1, the fabric was immersed in a monomer solution of glycidyl methacrylate/methanol (3/7) and

was allowed to react at 45°C for 3.5 h. Consequently, fibers having a methacrylic acid graft rate of 163% were obtained. These fibers were immersed in a 50% aqueous ethylenediamine solution at 50°C for 3 h, thus making the grafted fibers with amino groups.

Benamicin A, 5 mg, was dissolved in dimethyl sulfoxide, 0.4 ml, and the water-soluble carbodiimide hydrochloride, 21.5 mg, and n-propanol, 0.5 ml, were added and mixed. Then, five pieces of square sections of the grafted fibers with amino groups were soaked throughout in it, and the immobilization treatment was conducted at room temperature overnight.

After the reaction was over, the sections of the benamicin A-immobilized grafted fibers with amino groups were washed with n-propanol repeatedly until the washed solution turned colorless, making a sample of the benamicin A-immobilized grafted fibers with amino groups. Employing the rapid evaluation method for antibiotic activities that relied on Candida albicans 3143 as the test bacterium, the anti-yeast activity of the benamicin A-immobilized grafted fibers with amino groups was investigated in the coexistence or absence of calcium carbonate, 1 mg/ml, which is shown in FIG. 4. Similarly to the case of free benamicin A, the aniti-yeast activity of the immobilized benamicin was observed only in the coexistence of calcium ion.

Similarly to the case of free benamicin A, the anti-yeast activity of the immobilized benamicin was also inhibited when mannan or D-mannose coexisted. Particularly, it was markedly inhibited when D-mannose coexisted.

<u>Example 10 Grafted fibers with carboxyl groups Having Immobilized Streptomycin, Minocyclin, Chloramphenicol, or Macrolides (Erythromycin, Leucomycin, or Oleandomycin)</u>

Substituting ampicillin in the reaction composition according to Example 1 with an equal amount of streptomycin, minocyclin, chloramphenicol, erythromycin, leucomycin, jonasamycin, or oleandomycin, immobilization treatment was conducted similarly. After sufficient washing, antimicrobial activity against Staphylococcus aureus FDA209P was investigated in a similar manner to Example 1, and the manifestation of the antimicrobial activity was confirmed. The linking positions of the immobilized antibiotics have not yet been determined.

Structural formulae of the antibiotics used in Examples 9 and 10 are shown below.

1. Chloramphenicol
2. Streptomycin
3. Erythromycin
4. Minocyclin
5. Benanomicin

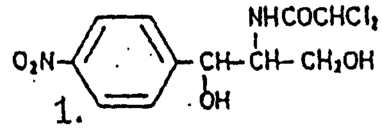

1.

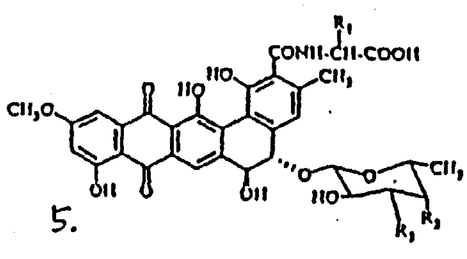

5.

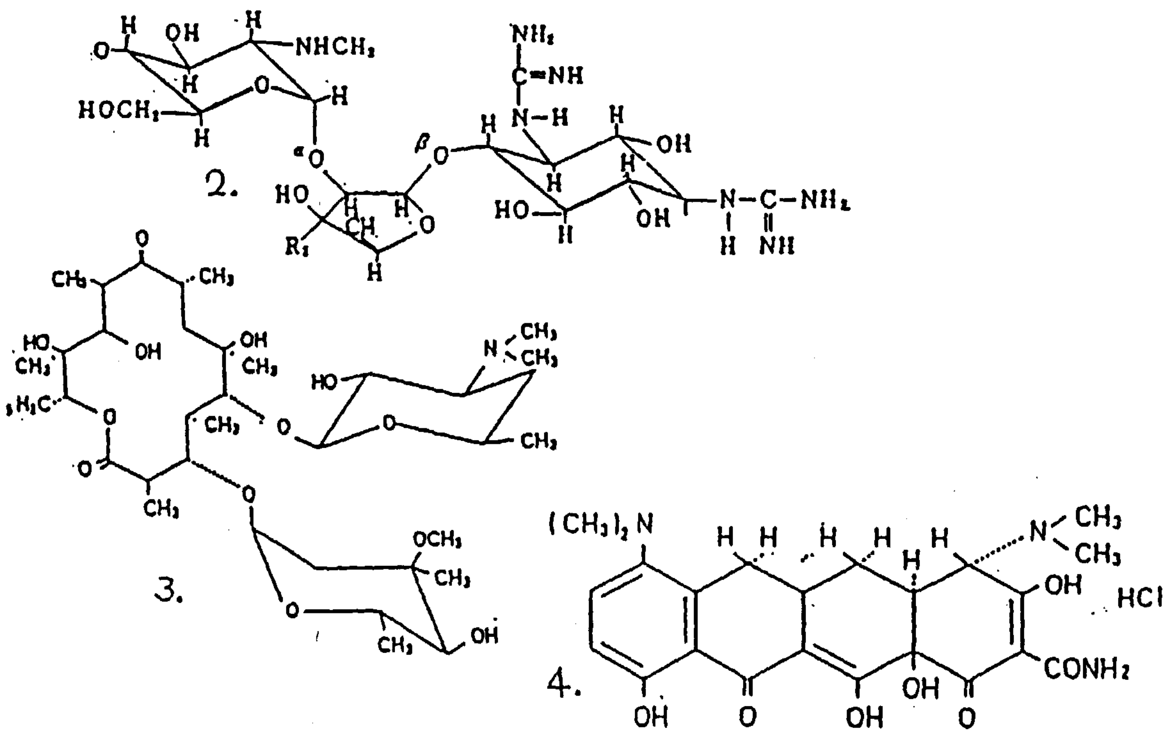

2.

3.

4.

The polymer products obtained by the methods of this invention can exert biological activities by directly contacting targets, without liberating drugs in their environments. Therefore, they neither give adverse influence on the agents other than the targets, nor induce the environmental contamination due to residual drugs, and they can provide methods of cure or treatment that has little residual adverse influence on humans, animals and plants, and the natural environment which should enjoy the results of drug use.

When grafted polymers are used as the polymer substrate, biologically active compounds can be linked to graft chains at the rate of one or more compounds per one graft chain by utilizing the graft chains as spacers; therefore, products that can exert great effects can be obtained.

## Claims

**1.** A biologically active polymer product having:

a polymer substrate; and

a biologically active compound moiety having low molecular weight, the portion being covalently bonded to the polymer substrate and exerting selective biological activity,

wherein the biologically active compound moiety exerts the selective biological activity while being covalently bonded to the polymer substrate.

2. The biologically active polymer product according to claim 1, wherein the polymer substrate comprises an organic polymer or an inorganic polymer.

3. The biologically active polymer product according to claim 1 or claim 2, wherein a graft chain is linked to the surface of the polymer substrate and the biologically active compound moiety is chemically linked to the graft chain.

4. The biologically active polymer product according to claim 3, wherein the biologically active compound moiety is chemically linked to the graft chain through an amide linkage or an amine linkage.

5. The biologically active polymer product according to any one of claims 1-4, wherein the biologically active compound moiety is chemotherapeutic.

6. The biologically active polymer product according to claim 5, wherein the chemotherapeutic is an antibiotic.

7. The biologically active polymer product according to claim 6, wherein the antibiotic is at least one antibiotic selected from a group of beta lactam antibiotics.

8. The biologically active polymer product according to claim 6, wherein the antibiotic is at least one antibiotic selected from a group of benzonaphthacenequinone antibiotics.

9. The biologically active polymer product according to claim 6, wherein the antibiotic is at least one antibiotic selected from the group consisting of tetracycline antibiotics, chloramphenicol antibiotics, macrolide antibiotics, and aminoglycoside antibiotics.

10. The biologically active polymer product according to any of the proceeding claims, wherein the biologically active compound moiety has a molecular weight not more than 5,000.

11. The biologically active polymer product according to any of the proceeding claims, wherein the biologically active compound moiety has a molecular weight not more than 2,000.

12. A biologically active polymer product comprising:

a polymer substrate;
two or more graft chains being linked to the polymer substrate;
pendant chains being branched from the respective graft chains; and
two or more biologically active compound moietys each being linked to each pendant chain and exerting biological activity selectively.

13. The biologically active polymer product according to claim 12, wherein the biologically active compound moiety is linked to the pendant chain through an amide linkage or an amine linkage.

14. A method for producing a biologically active polymer product, the product having:

a polymer substrate comprising a synthetic, organic polymer material;
a graft chain being linked to the surface of the polymer substrate; and
an antibiotic portion being chemically linked to the graft chain,
the method having:
the step of irradiating the polymer substrate that comprises the synthetic, organic polymer material with electron beam to provide an active site for graft formation;
the step of exposing a monomer to the polymer substrate so as to form two or more graft chains linked to the surface of the polymer substrate; and
allowing an antibiotic to react with the polymer substrate having the graft chain to form the antibiotic portion being chemically linked to the graft chain.

15. The method according to claim 14, wherein the antibiotic has a first functional group and the graft chain has a second functional group, wherein the first functional group reacts with the second functional group to be able to form an amide group, and the reaction step is carried out in the presence of a condensing agent.

**16.** The method according to claim 14, wherein the monomer in a gas-phase state is exposed to the polymer substrate.

**17.** A biologically active polymer product having:

a polymer substrate;
two or more graft chains being linked to the surface of the polymer substrate; and
an antibiotic portion being linked to the graft chain through an amido linkage or an amino linkage, the portion represented by the following formula:

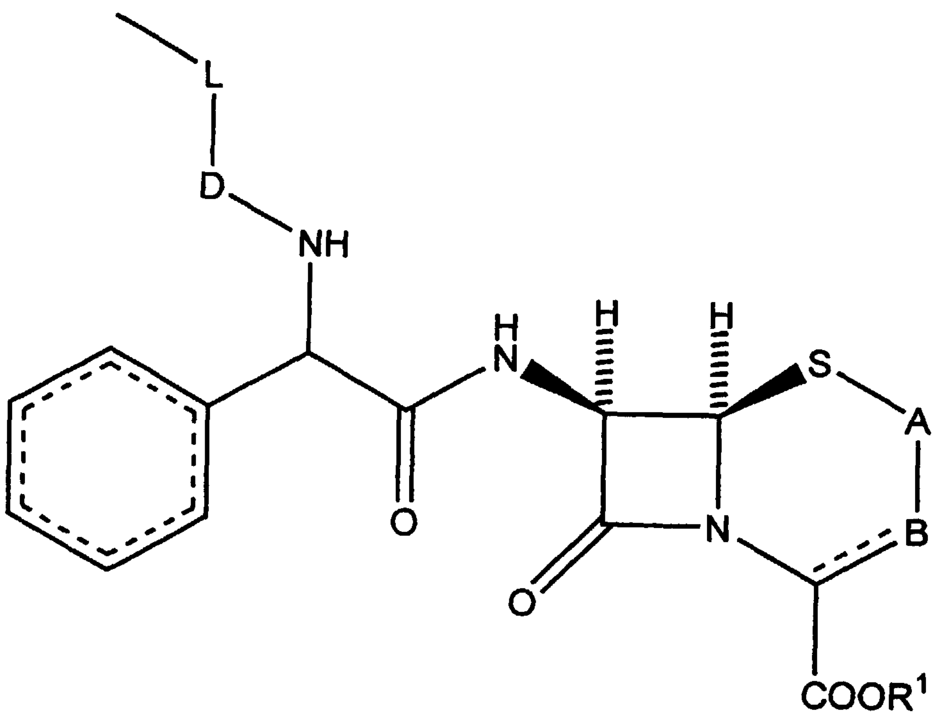

wherein L represents the graft chain portion being linked to the surface of the polymer substrate;
$R^1$ is a hydrogen atom, a cation, or a group represented by the following formula:

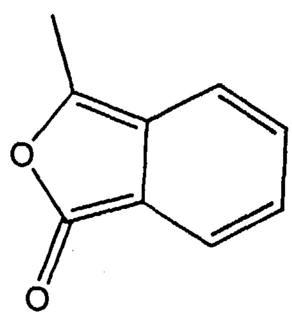

,

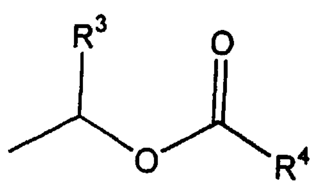

wherein $R^3$ and $R^4$ each independently represents a hydrogen atom, or a straight- or branched-lower alkyl group or a straight- or branched-lower alkoxy group each having 6 or less carbons, with the proviso that $R^3$ and $R^4$, together, may form a saturated or unsaturated 5- to 7-membered ring and a benzene ring may further be fused to the 5- to 7-membered ring;
"A" represents no bond or a methylene group;
"B" is a group represented by formula: $C(R^5)$, or a group represented by the formula: $C(R^5)_2$, wherein $R^5$ is a lower alkyl group having 6 or less carbons, or a group represented by the formula: $-CH(R^6)O-C(=O)R^7$, wherein $R^6$ is a hydrogen atom or a lower alkyl group having 6 or less carbons and $R^7$ is a lower alkyl group having 6 or less carbons;

"D" represents no bond or a carbonyl group; and

- - - represents a single bond or a double bond.

**18.** The biologically active polymer product according to claim 17, wherein the antibiotic portion is linked to the graft chain through a pendant chain.

**19.** The biologically active polymer product according to claim 17, wherein the hydrocarbon 6-membered ring in the formula is a benzene ring and $R^5$ is a methyl group.

**20.** The biologically active polymer product according to claim 17, wherein "D" is a carbonyl group.

**21.** The biologically active polymer product according to claim 17, wherein "A" is non-bond.

**22.** The biologically active polymer product according to claim 17, wherein "A" is a methylene group.

Fig. 1A

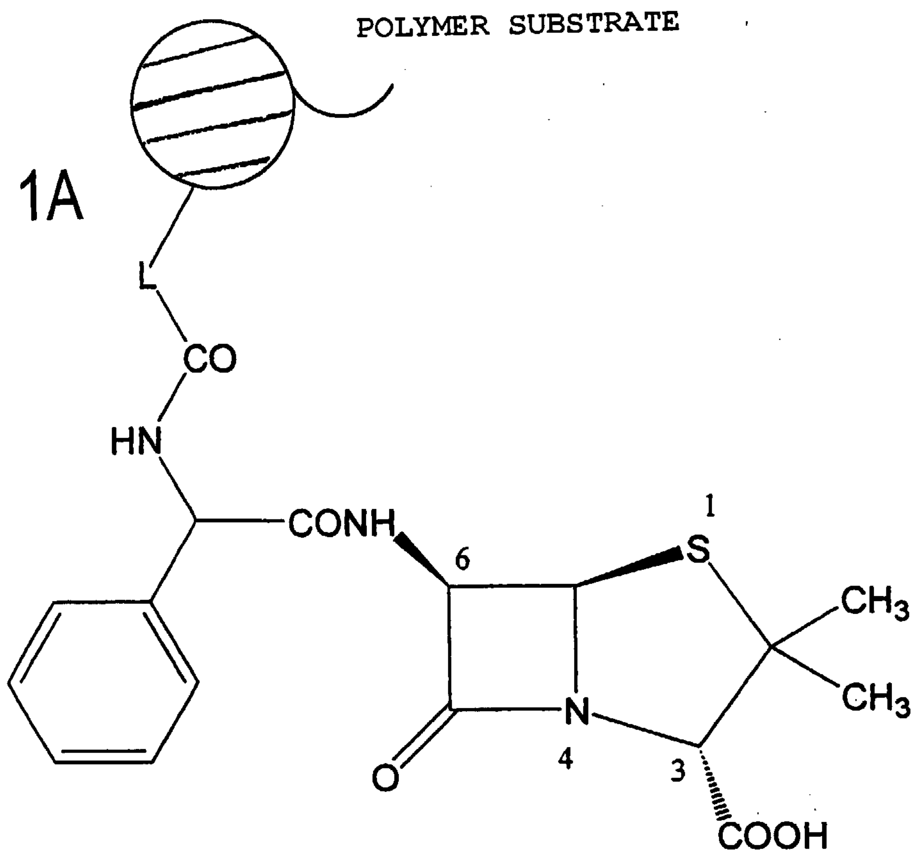

Fig. 1B

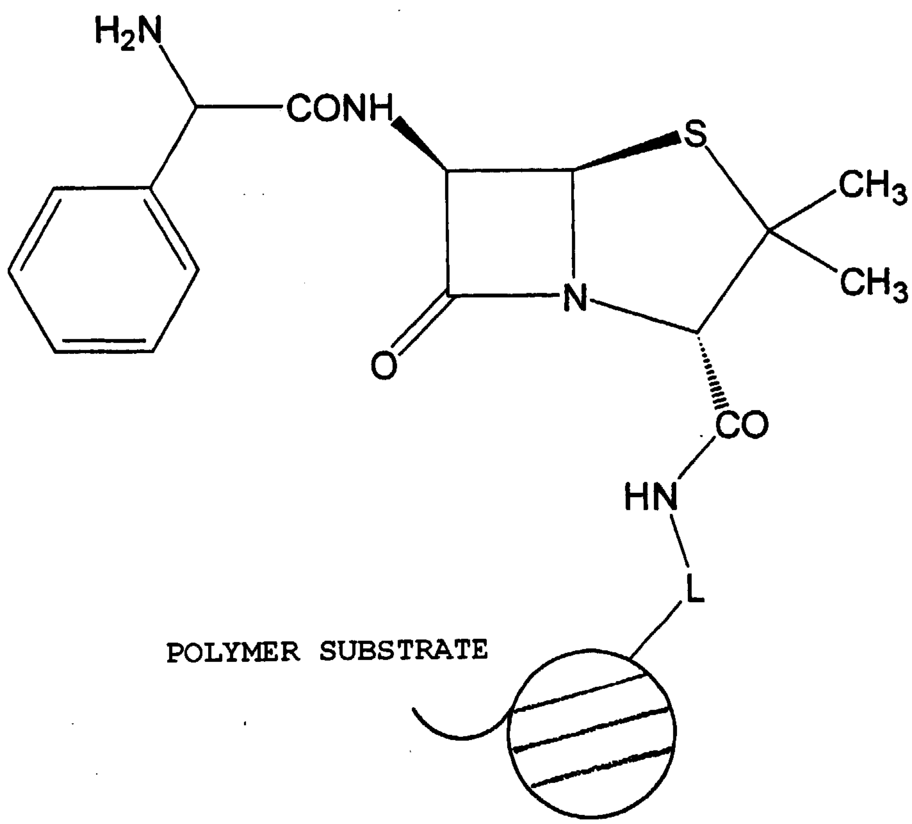

Fig. 2

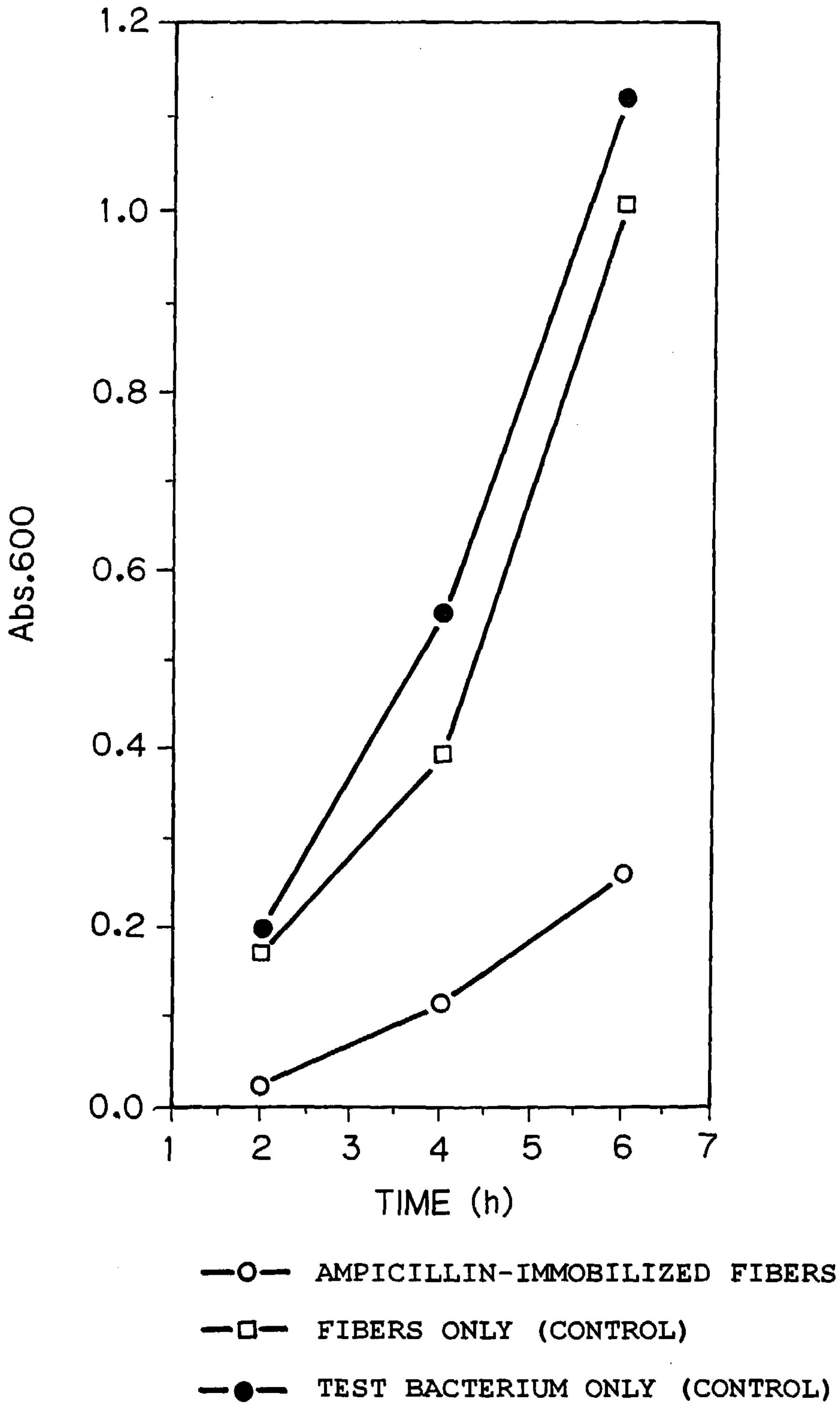
1.2
1.0
0.8
0.6
0.4
0.2
0.0
Abs.600
1
2
3
4
5
6
7
TIME (h)
AMPICILLIN-IMMOBILIZED FIBERS
FIBERS ONLY (CONTROL)
TEST BACTERIUM ONLY (CONTROL)

Fig. 3

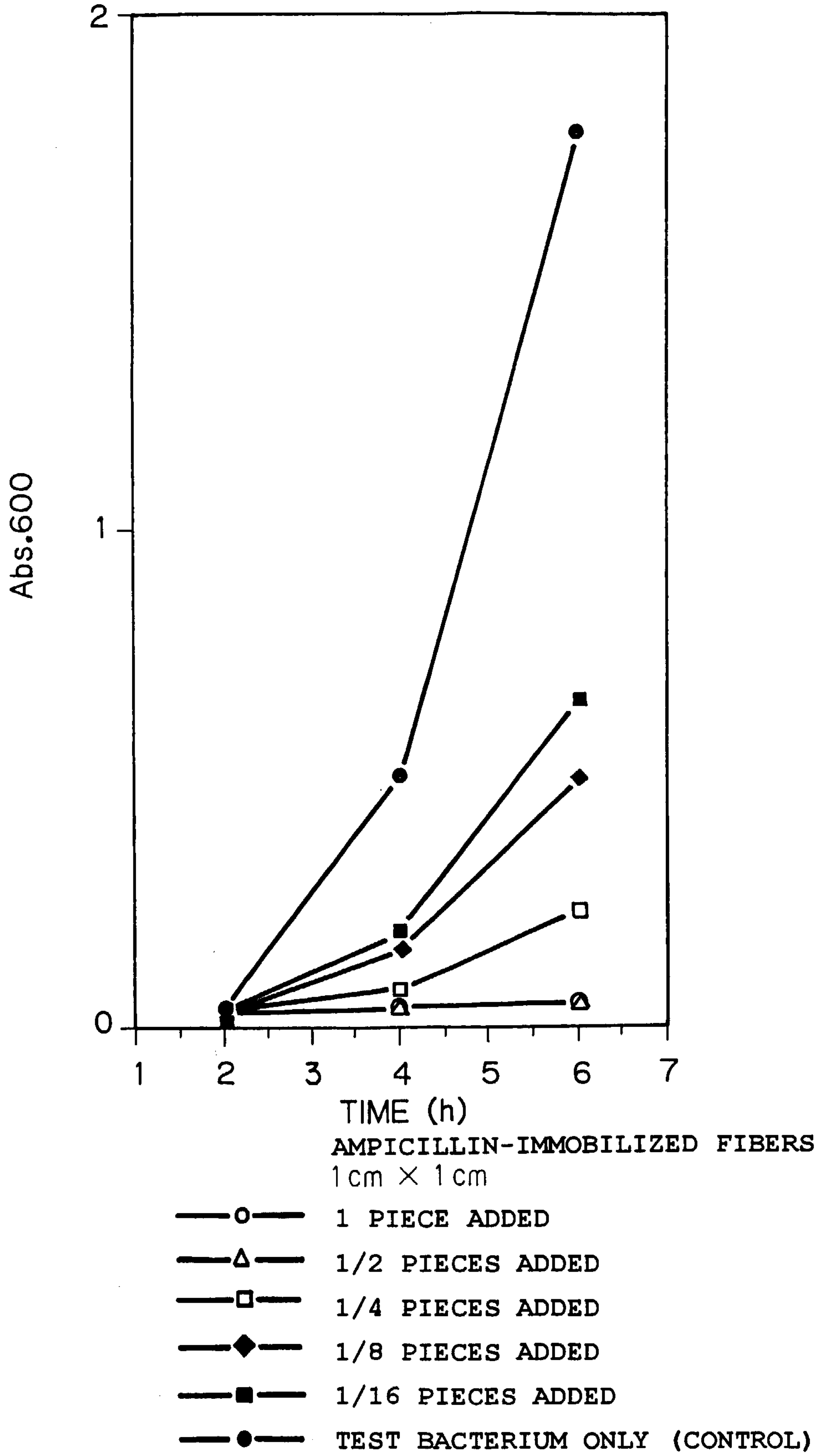

2
1
0
Abs.600
1
2
3
4
5
6
7
TIME (h)
AMPICILLIN-IMMOBILIZED FIBERS
1cm × 1cm
1 PIECE ADDED
1/2 PIECES ADDED
1/4 PIECES ADDED
1/8 PIECES ADDED
1/16 PIECES ADDED
TEST BACTERIUM ONLY (CONTROL)

Fig. 4

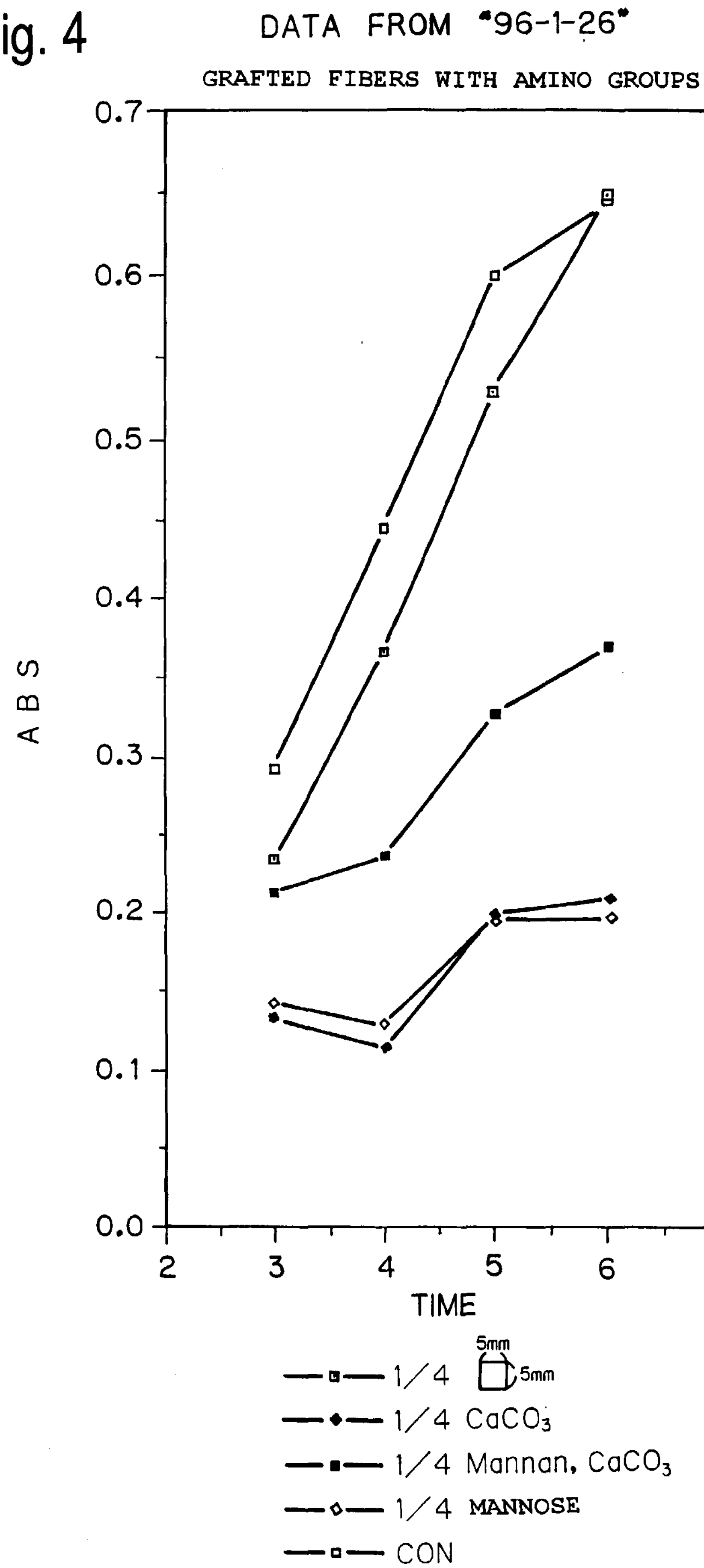
DATA FROM "96-1-26"
GRAFTED FIBERS WITH AMINO GROUPS
0.7
0.6
0.5
0.4
0.3
0.2
0.1
0.0
ABS
2
3
4
5
6
7
TIME
1/4 5mm 5mm
1/4 CaCO3
1/4 Mannan, CaCO3
1/4 MANNOSE
CON

Fig. 5B

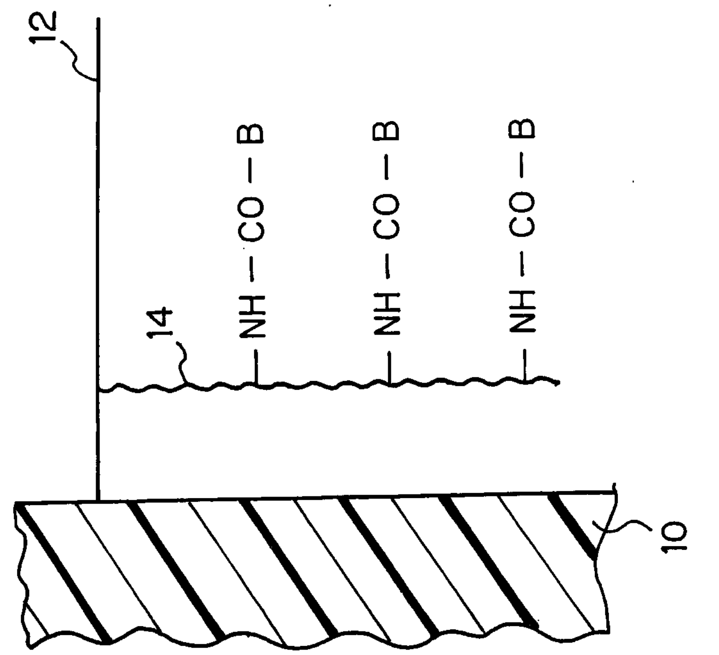


Fig. 5A

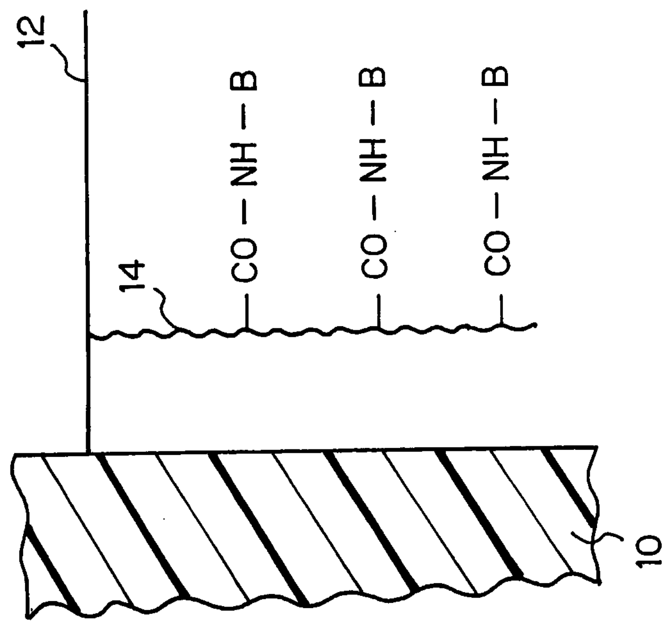

Fig. 6

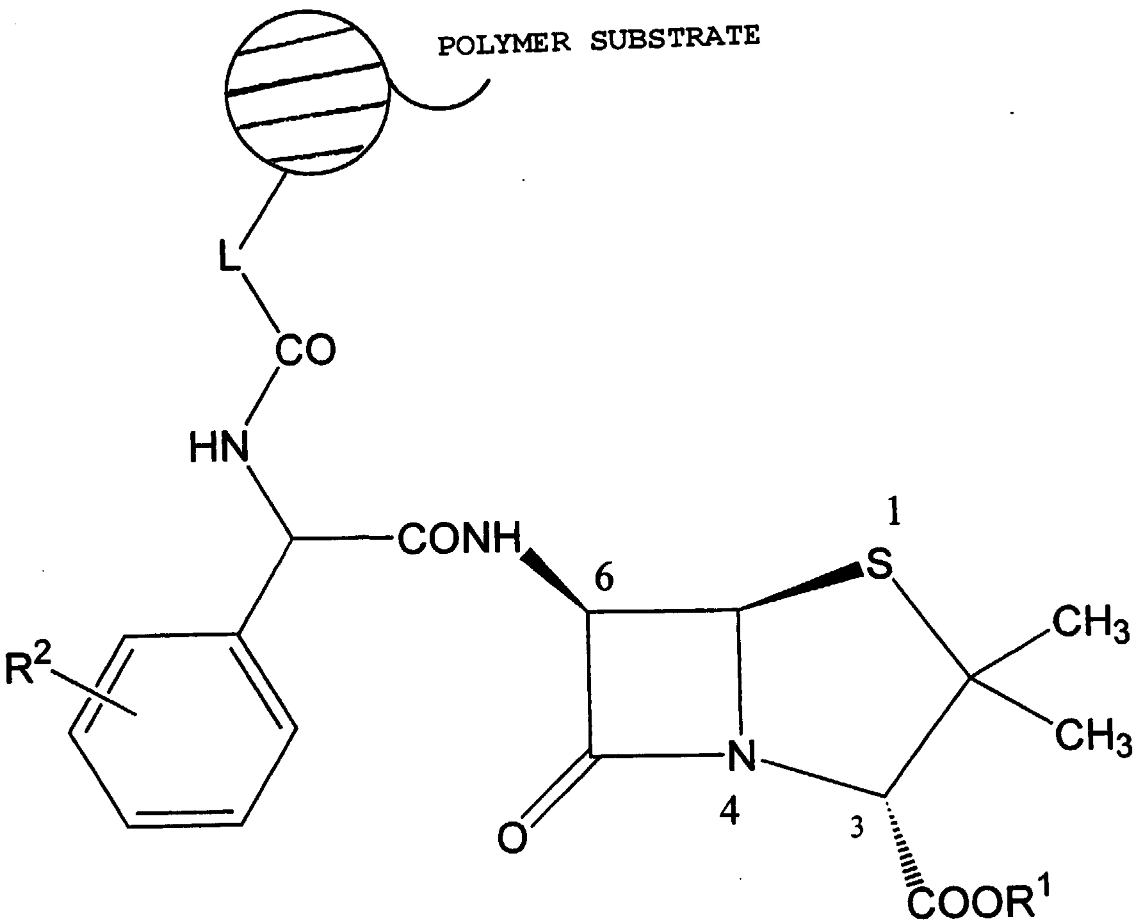

Fig. 7

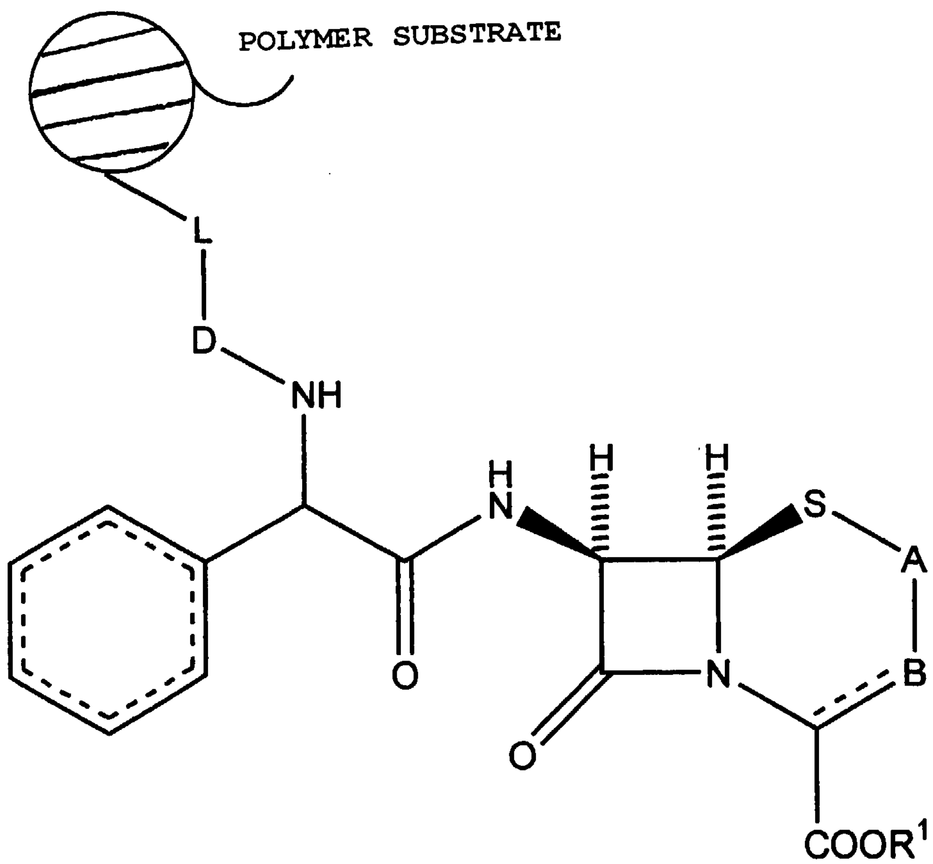

## INTERNATIONAL SEARCH REPORT

International application No. PCT/JP97/03710

**A. CLASSIFICATION OF SUBJECT MATTER**

Int. $Cl^6$ A61K47/48

According to International Patent Classification (IPC) or to both national classification and IPC

**B. FIELDS SEARCHED**

Minimum documentation searched (classification system followed by classification symbols)

Int. $Cl^6$ A61K47/48

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP, 3-17025, A (NOF Corp.), January 25, 1991 (25. 01. 91) (Family: none) | 1 - 22 |
| A | JP, 3-287545, A (Research Development Corp. of Japan), December 18, 1991 (18. 12. 91) (Family: none) | 1 - 22 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| November 26, 1997 (26. 11. 97) | December 9, 1997 (09. 12. 97) |
| Name and mailing address of the ISA/ Japanese Patent Office | Authorized officer |
| Facsimile No. | Telephone No. |

Form PCT/ISA/210 (second sheet) (July 1992)